# EUROPEAN PATENT APPLICATION

(11) **EP 4 714 969 A1**
(43) Date of publication of application: **25.03.2026**
(21) Application number: 24382996.7
(22) Date of filing: 19.09.2024
(51) Int. Cl.: C07K 16/28, A61K 38/17, A61P 31/00, A61P 35/00

(54) **COMPOSITIONS COMPRISING SOLUBLE TIGIT AND PD-1 - PD-L1 AXIS INHIBITORS AND USES THEREOF**

(71) Applicant: Fundació Privada Institut de Recerca Sobre Immunopatologies-Caixa Irsicaixa, 08916 Badalona (ES)
(72) Inventor: GARCÍA PRADO, Julia, E-08916 Badalona (ES); MARÍN LÓPEZ, Miguel Ángel, E-08916 Badalona (ES); LÁZARO DÍEZ, María, E-08916 Badalona (ES)
(74) Representative: ABG Intellectual Property Law, S.L.

(57) **Abstract**

The invention relates to the field of immunotherapeutics and, more particularly, to a composition based on the combination of a TIGIT variant and an inhibitor of the PD-1/PD-L1 axis and its use for treating or delaying an immune-related disease.

## Description

### FIELD OF THE INVENTION

The invention relates to the field of immunotherapeutics, concretely to the field of immunity restoration.

### BACKGROUND ART

T cells are immune cells that become activated via T cell receptor (TCR) signaling following engagement with antigen. Physiologic activation through the T cell receptor renders T cells capable of mediating potent antitumor or anti-infective effects. During resolution of an acute inflammatory response, a subset of activated effector T cells differentiate into long-lived memory cells. By contrast, in patients with chronic infections or cancer, T cells not infrequently undergo pathologic differentiation toward a state of dysfunction, which has been termed immune exhaustion.

Immune exhaustion, particularly T cell exhaustion, which is an acquired state of T cell dysfunction, is a hallmark of cancer and chronic viral infection. T cell exhaustion is characterized by marked changes in metabolic function, transcriptional programming, loss of effector function (e.g., cytokine secretion, killing capacity), and co-expression of multiple surface inhibitory receptors. The root cause of T cell exhaustion is persistent antigen exposure leading to continuous TCR signaling.

Accordingly, there is a need in the art to provide means to reduce T cell exhaustion as means to enhance immune effectiveness in patients with cancer or chronic infections.

### SUMMARY OF THE INVENTION

The authors of the present invention have developed compositions comprising soluble forms of TIGIT and an inhibitor of the PD-1/PD-L1 axis that may activate the immune system in the context of dendritic and T-cells and restore immune function lost by the immune exhaustion. Thus, these compositions may be useful in treating and delaying immune-related diseases.

Thus, in a first aspect, the present invention relates to a composition, hereinafter "the composition of the invention" comprising:
a) a first component selected from the group consisting of:
   i) A polypeptide comprising a TIGIT variant that comprises the TIGIT Ig-like V-type domain and wherein said polypeptide lacks the TIGIT transmembrane and cytoplasmic domains and,
   ii) a polynucleotide encoding the polypeptide defined in i),
   and
b) a second component, which is an inhibitor of the PD-1/PD-L1 axis.

In a second aspect, the present invention relates to a pharmaceutical composition, hereinafter "the pharmaceutical composition of the invention" comprising a pharmaceutically effective amount of the composition of the invention and a pharmaceutically acceptable excipient.

In a third aspect, the present invention relates to the composition of the invention or the pharmaceutical composition of the invention for use in medicine.

In a fourth aspect, the present invention relates to the composition of the invention of the pharmaceutical composition of the invention for use in a method for treating or delaying an immune-related disease.

In a fifth aspect, the present invention relates to a polypeptide comprising a TIGIT variant that comprises the TIGIT Ig-like V-type domain and wherein said polypeptide lacks the TIGIT transmembrane and cytoplasmic domains or a polynucleotide encoding said polypeptide for use in a method for reducing immune toxicity in a patient that is being treated with an inhibitor of the PD-1/PD-L1 axis.

### DESCRIPTION OF THE FIGURES

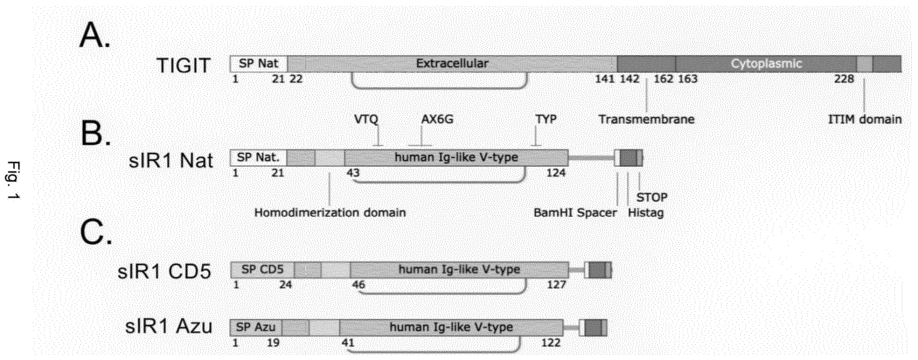

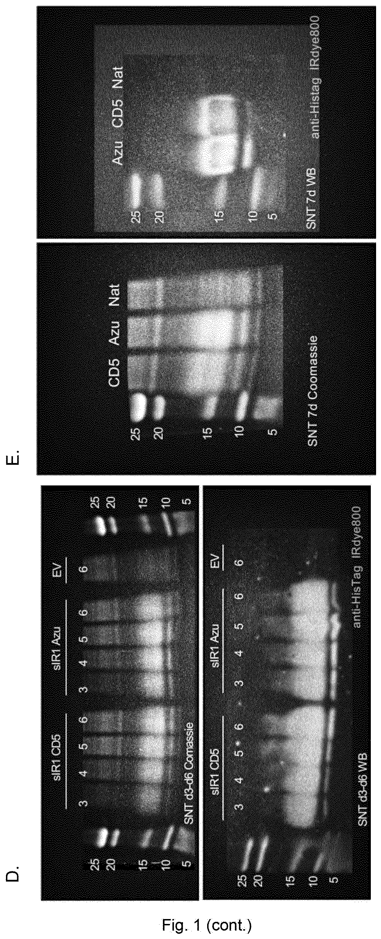

**Figure 1****: sIR1 protein structure, in silico modelling and protein production. A.** Structure of human TIGIT pre-protein containing the native signal peptide (SP Nat) (cleaved during intracellular processing), the extracellular, the transmembrane and the cytoplasmatic region. **B.** Structure of slR1 Nat contains the SP Nat and the extracellular region of TIGIT. The Ig-like V-Type domain includes the regions for homodimerization and interaction with CD155/PVR (VTQ, AX6G, and TYP). For solubilization, the transmembrane sequences of TIGIT were removed, and sequences for cloning (BamHI), detection (His-tag) and protein termination (STOP) were included. C. Signal peptide optimization was performed on slR1 Nat by incorporating the SP from human CD5 preproprotein (SP CD5) or Azurocidin preproprotein (SP Azu) in slR1 CD5 and Azu, respectively. Moreover, the tail sequence following the Ig-like V-Type was shortened to avoid retention in the membrane. **D.** Supernatants from transfection were collected after 7 days and analysed by SDS-PAGE with Coomassie staining (left) or WB (right) to perform a protein production screening. **E.** Time course analysis performed by Coomassie staining (top) or WB (bottom) in supernatants collected 3-6 days post-transfection. Supernatant (SNT), Empty vector (EV), western blot (WB).

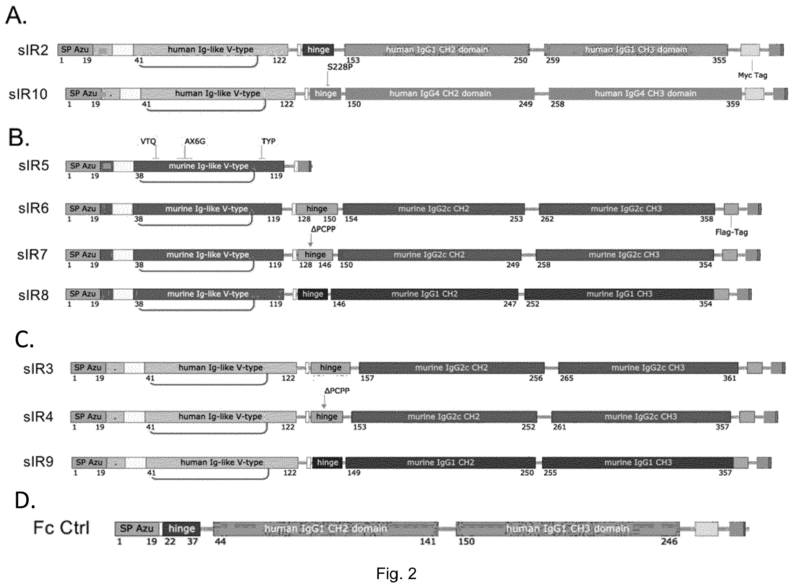

**Figure 2****: siR protein structure of human, murine and hybrid stable dimers prototypes. A.** Human sIR prototypes. Structure of dimeric slR2 containing human effector IgG1 Fc domain and slR10 containing human no effector IgG4 Fc domain with S228P mutation. Human stable dimers contain Myc-tag and His-tag **B.** Murine sIR prototypes. Structure of monomeric slR5 containing murine the TIGIT Ig-like V- type domain, dimeric slR6 containing murine effector IgG2c Fc domain, dimeric sIR7 containing murine effector IgG2c Fc domain with a ΔPCPP (SEQ ID NO: 45) deletion, and dimeric slR8 containing murine no effector IgG1 Fc domain with a ΔPCPP. Murine stable dimers contain Flag-tag and His-tag. **C.** Hybrid sIR prototypes. Structure of dimeric slR6 containing murine effector IgG2c Fc domain, dimeric sIR7 containing murine effector IgG2c Fc domain with a ΔPCPP deletion, and dimeric slR8 containing murine no effector IgG1 Fc domain with a ΔPCPP. **D.** Control recombinant protein generated with the deletion of Ig-like V-type domain form sIR2. Signal peptides depicted as SP in the different prototypes are cleaved during intracellular processing and are not present in the final mature secreted products.

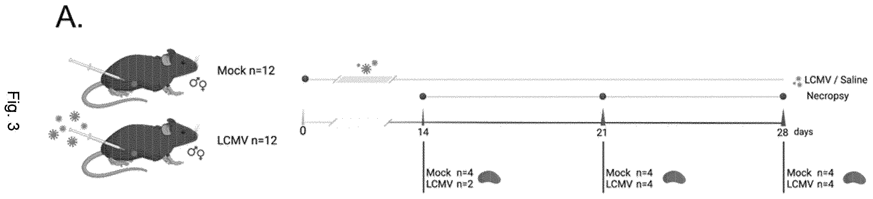

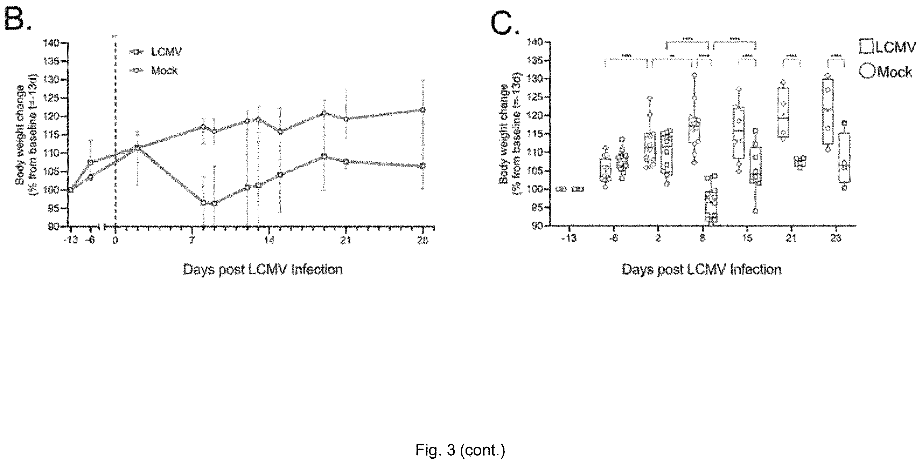

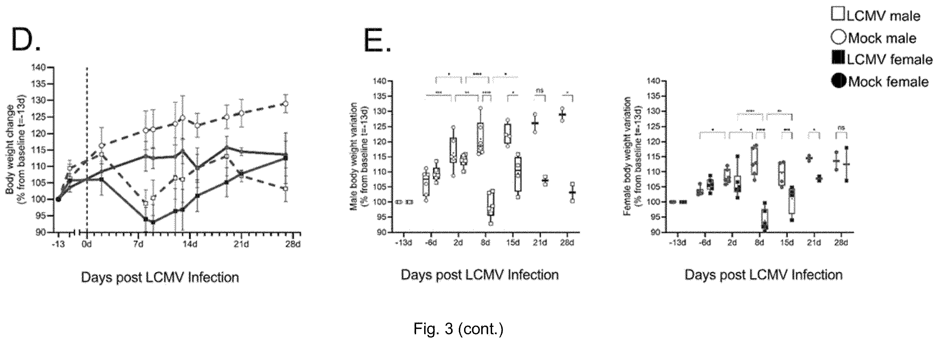

**Figure 3****: Body weight variation during LCMV infection. A.** Schematic representation of study design to characterize chronic LCMV_{DOC} infection in C57BU6 mice. The study included weight monitoring for 28 days and necropsy at three endpoints (14, 21 and 28 days) in Mock (n=12) and LCMV-infected mice (n=12). **B.** Median body weight variation from baseline (t=13 days) after IP administration of LCMV. **C.** Box Plots represent the median percentage of body weight variation from baseline ± IQR at -6, 2, 8, 15, 21 and 28-days post-infection. Each point represents a mouse. **D.** Median percentage of body weight variation from baseline (t=13 days) disaggregated by sex. **E.** Box Plots represent the median percentage of body weight variation from baseline

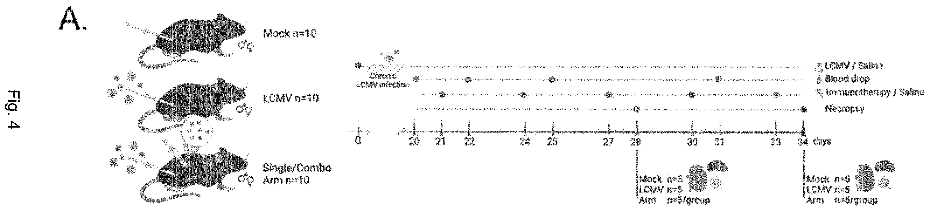

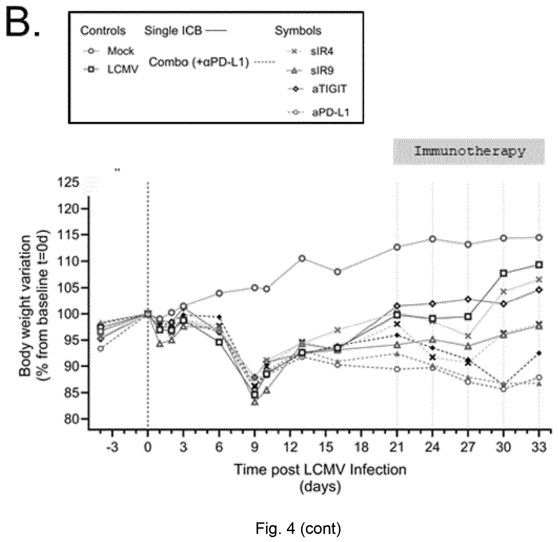

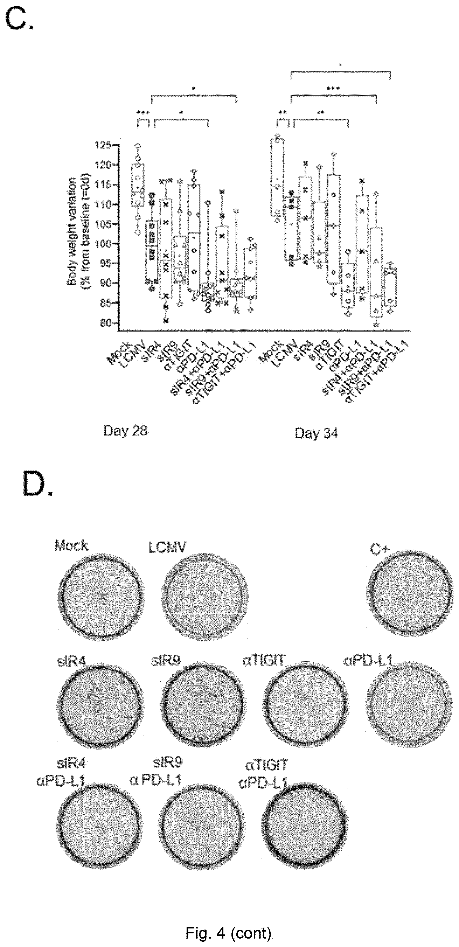

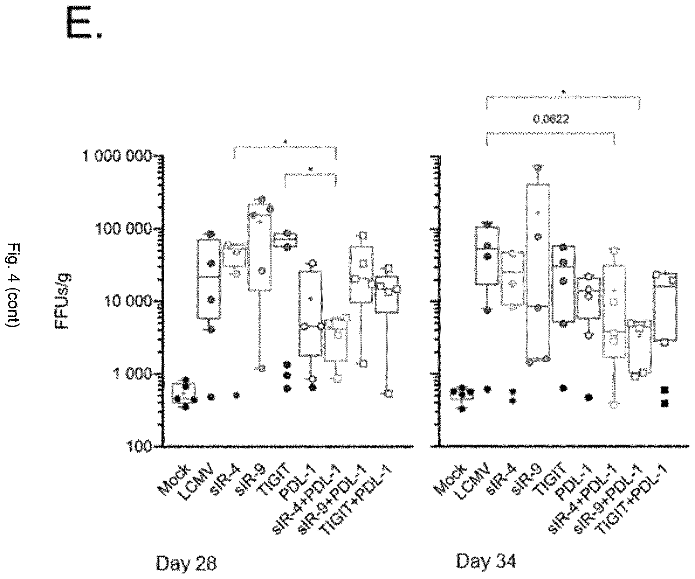

**Figure 4****: LCMV chronic infection in C57BL/6 mice efficacy study of TIGIT/CD155 blockade in combination with αPD-L1. A.** Schematic representation of study design. The study included weight monitoring and blood sampling for 34 days with two necropsy endpoints on days 28 and 34. **B.** Median body weight variation from baseline (t=0 days) after IP administration of LCMV. **C.** Box Plots represent the median percentage of body weight variation from baseline ± IQR at 27-days and 33-days post-infection. Each point represents a mouse. **D.** Representative LCMV viral load determination by FFU assay in MC57 cell lines from spleen samples. **E.** LCMV viral load (FFU/g). Undetectable samples are indicated with black dots in the limit of quantification. White symbols represent αPD-L1 blockade alone or in combination. Intraperitoneal (IP).

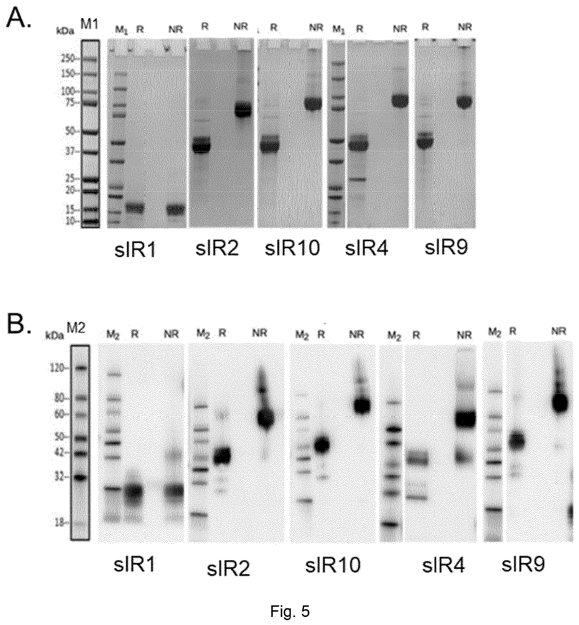

**Figure 5****: Protein characterisation and quality control. A.** Coomassie blue staining was performed on purified human sIRI, slR2 and sIR10, and hybrid slR4 and slR9 prototypes. The Coomassie blue was conducted under reducing (R) and non-reducing (NR) conditions. Marker 1 (M1) was used as a reference. **B.** Western blot analysis was performed on the purified human sIR1, sIR2, sIR10 prototypes, and hybrid slR4 and slR9 prototypes, detecting the His-tag located in the C-terminal domain. The analysis was conducted under reducing (R) and non-reducing (NR) conditions. Marker 2 (M2) was used as a reference.

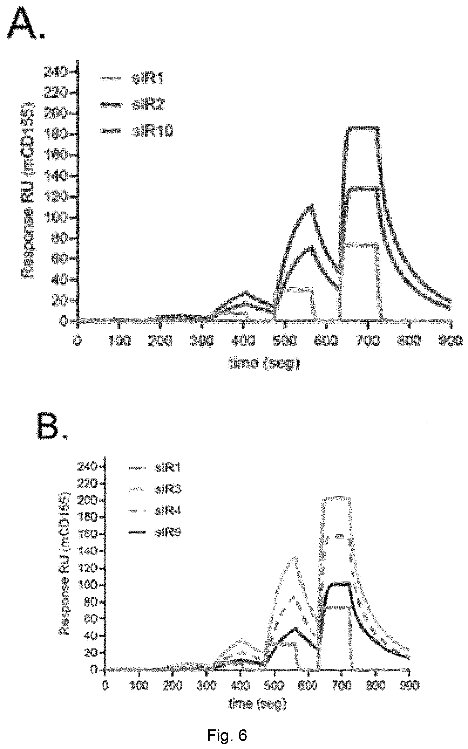

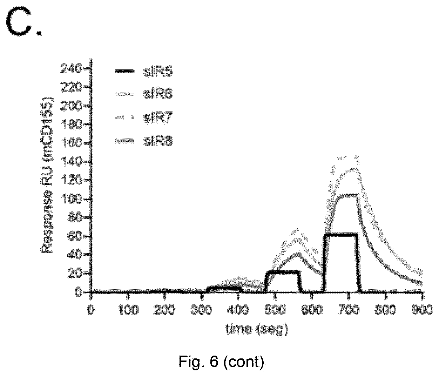

**Figure 6****: Protein characterisation and quality control. A.** Coomassie blue staining was performed on purified human sIRI, slR2 and sIR10, and hybrid slR4 and slR9 prototypes. The Coomassie blue was conducted under reducing (R) and non-reducing (NR) conditions. Marker 1 (M1) was used as a reference. **B.** Western blot analysis was performed on the purified human sIR1, sIR2, sIR10 prototypes, and hybrid slR4 and slR9 prototypes, detecting the His-tag located in the C-terminal domain. The analysis was conducted under reducing (R) and non-reducing (NR) conditions. Marker 2 (M2) was used as a reference.

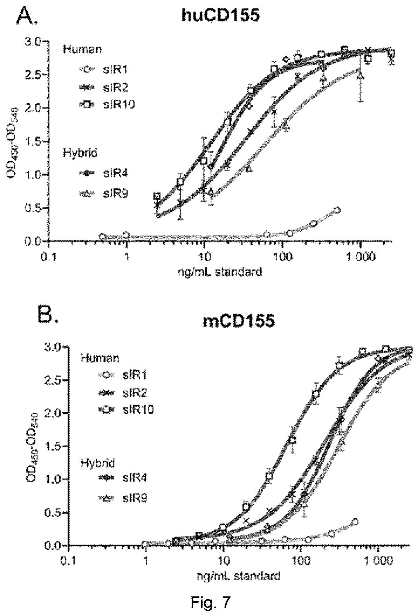

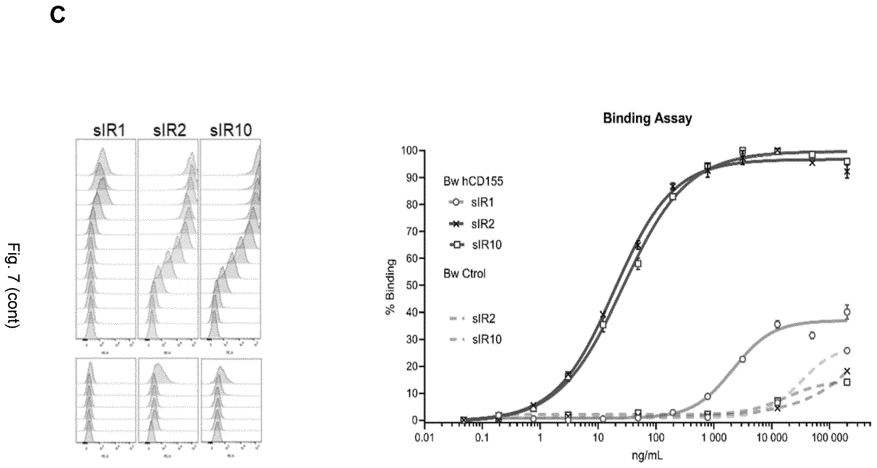

**Figure 7****: Binding of siRs to CD155 by functional ELISA and Bw5147 cell-based assay.** Functional ELISA to determine the IC₅₀ values of human sIRI, sIR2, and s!R10 and hybrid slR4 and slR9 was conducted on plates coated with hCD155 (**A.**) and mCD155 (**B.**). Data represent mean OD450-540 values from experimental duplicates. **C.** Bw hCD155 or Bw Ctrol cells were incubated with serial dilutions of sIR1, slR2 and sIR10 for 1 hour at 4°C. The binding of sIRs to hCD155 was assessed by flow cytometry using an anti-His-tag PE antibody (left panel). The percentage of binding of sIRs to hCD155 was determined in Bw cells by normalised log transform of PE MFI intensity. IC₅₀ was determined using a sigmoidal 4PL regression model.

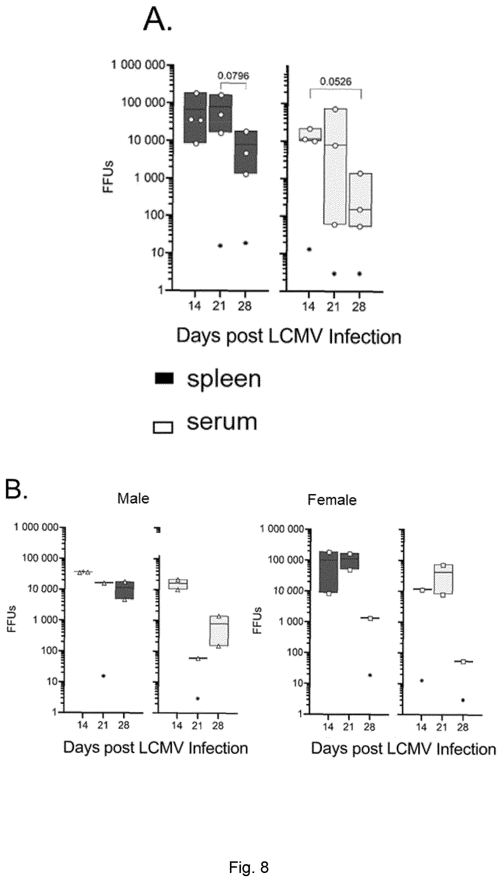

**Figure 8****: Viral persistence in spleen and serum after LCMV chronic infection. A.** LCMV viral load was determined by FFU assay in MC57 cell lines from spleen and serum samples on days 14, 21 and 28 post-infection. **B.** LCMV viral load disaggregated by sex. Undetectable samples are indicated with a black dot in the limit of quantification per sample.

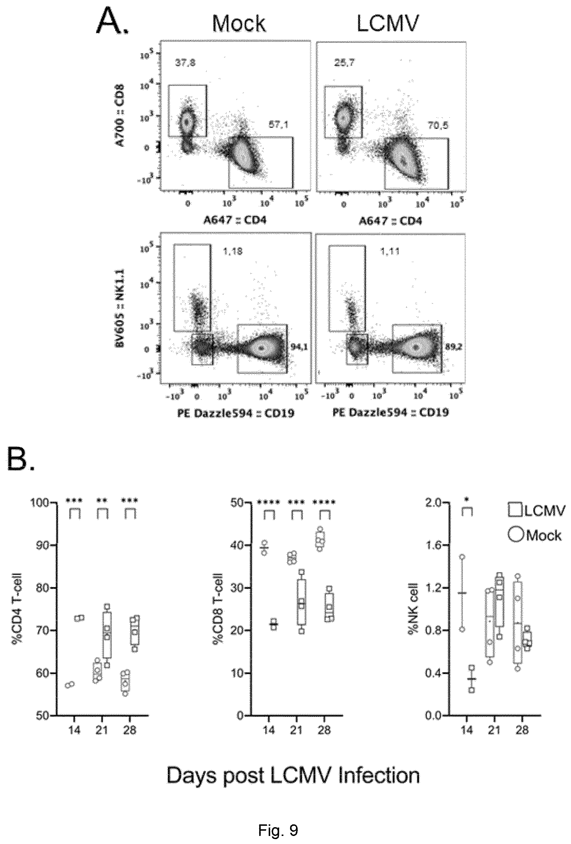

**Figure 9****: Lymphocytic cell populations are altered by LCMV chronic infection A.** Representative dot plots of CD8⁺, CD4⁺ T-cell gated on CD3+ splenocytes and NK cells gated on CD3- splenocytes **B.** Frequency of total CD8⁺ T-cells, CD4⁺ T-cells, and NK cells during LMCV chronic infection on day 14, 21, and 28 compared to uninfected mice (Mock).

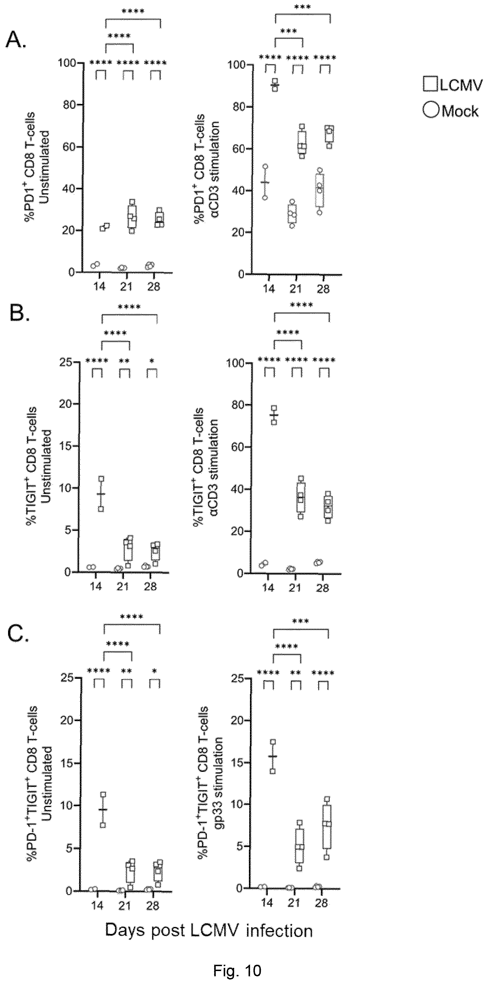

**Figure 10****: Expression of PD-1 and TIGIT in** CD8⁺ **T-cells during chronic LCMV infection. A.** Frequency of PD-1+CD8⁺ T-cells during LCMV chronic infection on day 14, 21, and 28 post-infection compared to uninfected (Mock) mice. Unstimulated (Left), αCD3 stimulation (right). **B.** Frequency of total TIGIT+CD8⁺ T-cells during LMCV chronic infection on day 14, 21, and 28 post-infection compared to uninfected (Mock) mice. Unstimulated (Left), αCD3 stimulation (right). **C.** Frequency of PD-1+TIGIT+ CD8⁺ T-cells during LMCV infection in response to gp33-specific stimulation on day 14, 21, and 28 post-infection compared to uninfected (Mock) mice.

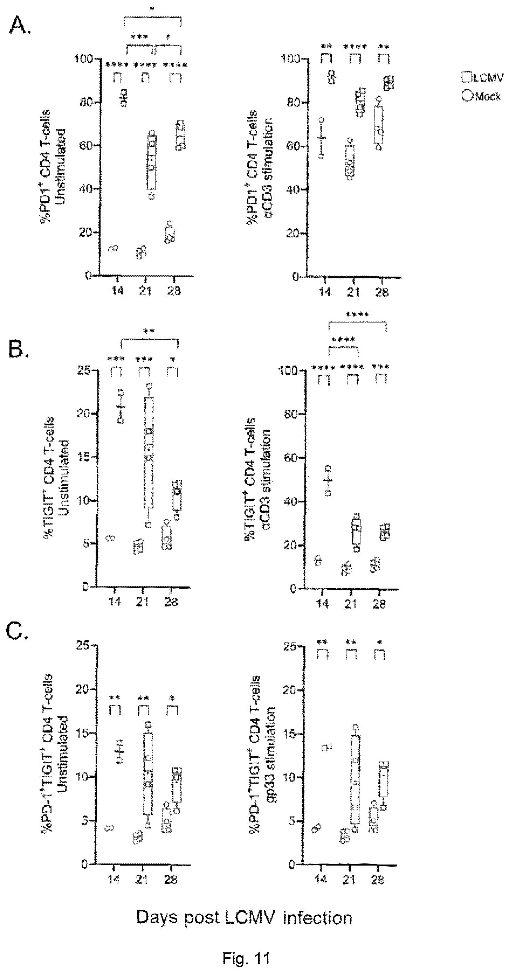

**Figure 11****: Expression of PD-1 and TIGIT in CD4⁺ T-cell during chronic LCMV infection. A.** Frequency of total PD- 1+CD4⁺ T-cells during LMCV chronic infection on day 14, 21, and 28 compared to uninfected (Mock) mice. Unstimulated (Left), αCD3 stimulation (right). **B.** Frequency of TIGIT+CD4⁺ T-cells during LMCV chronic infection on day 14, 21, and 28 compared to uninfected (Mock) mice. Unstimulated (Left), αCD3 stimulation (right). **C.** Frequency of PD-1 + TIGIT + CD4⁺ T-cells during LMCV infection in response to gp33-specific stimulation on day 14, 21, and 28 post-infection compared to uninfected (Mock) mice.

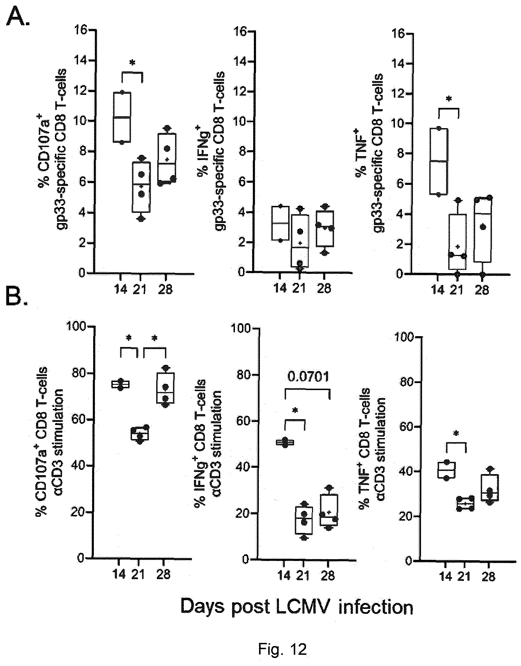

**Figure 12****: CD8⁺ T-cell responses during chronic LCMV infection. A.** Frequency of CD107a, IFNy and TNF production in CD8⁺ T-cell in αCD3 stimulation. **B.** Frequency of CD107a, IFNy and TNF gp33-specific CD8⁺ T-cells.

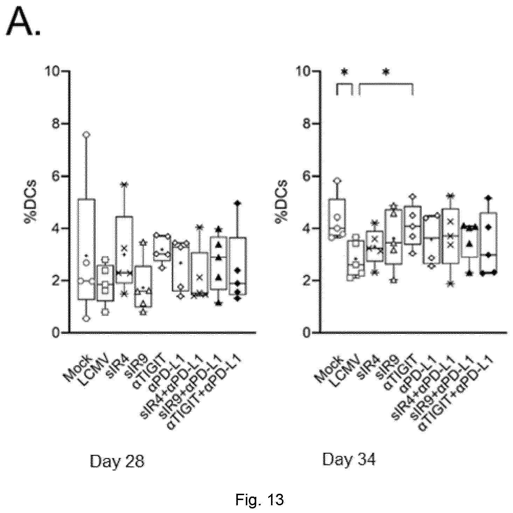

**Figure 13****: DCs are altered by chronic LCMV infection. A.** Frequency of DCs on day 28 and 34 post-infection.

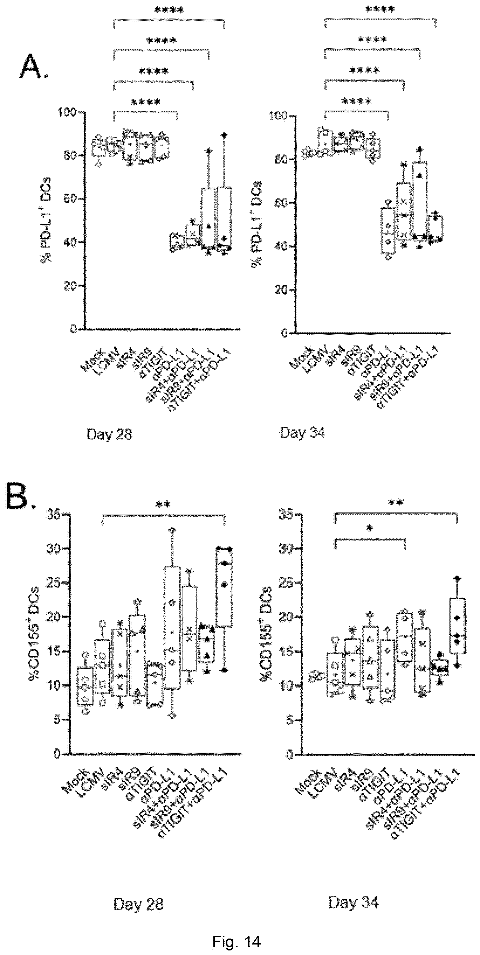

**Figure 14 B-D: Frequency of PD-L1 in DCs is decreased in single and combined αPD-L1 increasing the levels of DCs activation.** A-C. Frequency of total PD-L1+, CD155+ and CD40+ DCs on day 28 and 34 after LMCV infection across study arms.

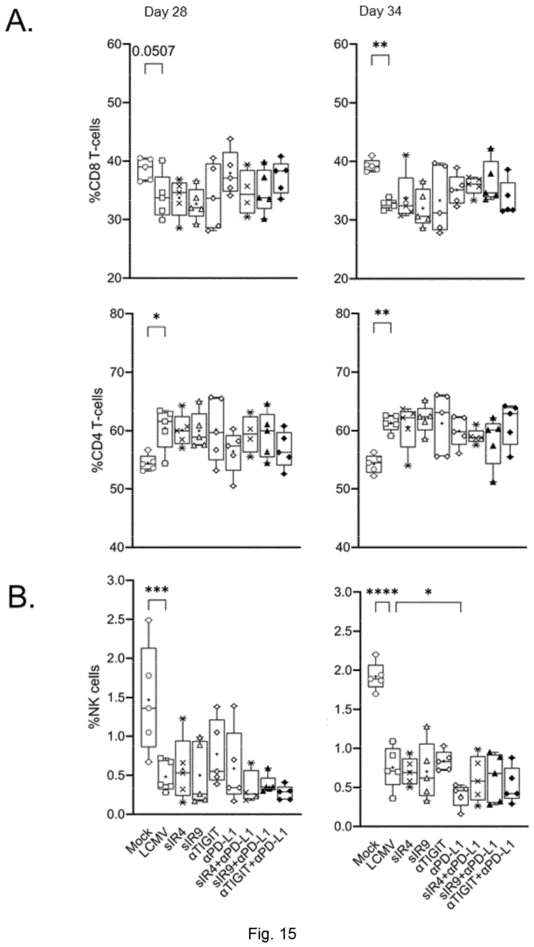

**Figure 15****: Lymphocytic and NK populations are altered by LCMV chronic infection and not normalized by immune interventions. A-B.** Frequency of total CD8+ T-cells, CD4+ T-cells, and NK cells at days 28 and 34 after infection.

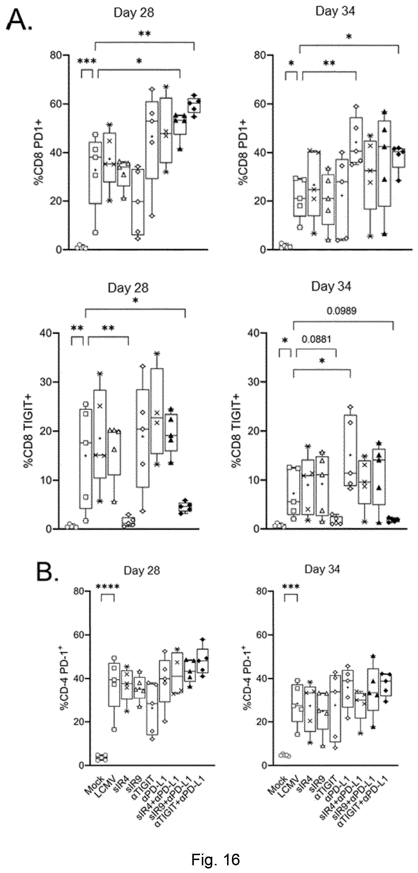

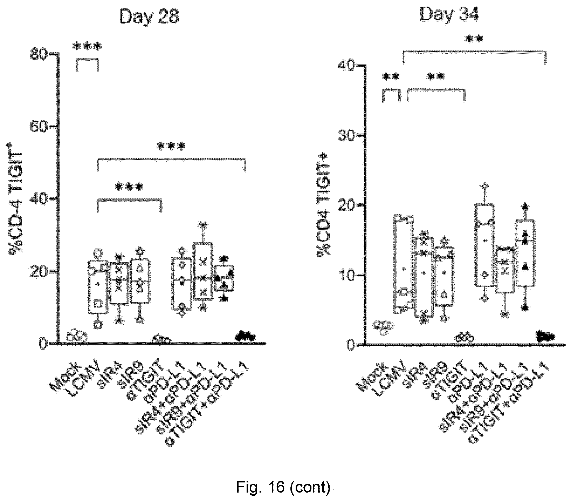

**Figure 16****: Expression levels of PD-1 and TIGIT in CD8⁺ and CD4⁺ T cells in LCMV chronic infection. A-B.** Frequency of PD-1 and TIGIT expression in CD8⁺ T-cells, CD4⁺ T-cells on day 28 and 34.

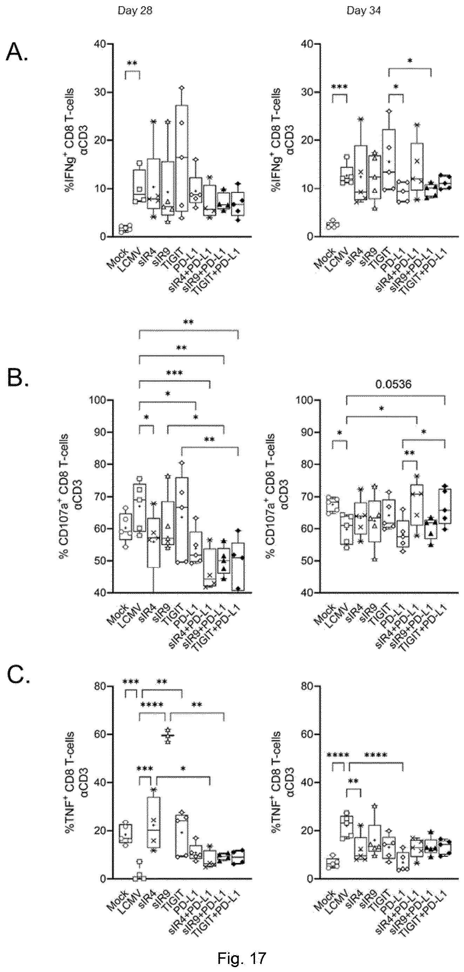

**Figure 17****: CD8⁺ T-cell function in αCD3 stimulated condition. A-C.** Frequency of IFNγ, CD107a, and TNF in CD8⁺ T-cells.

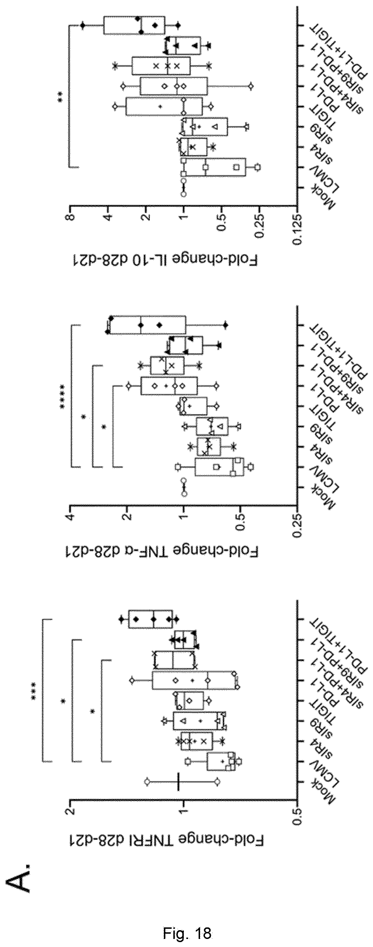

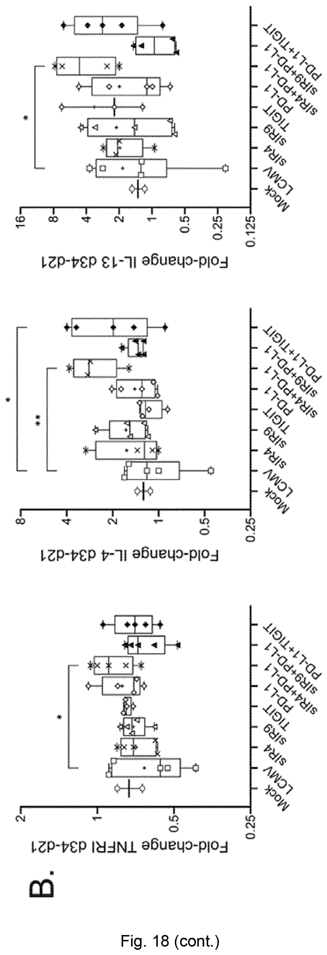

**Figure 18****: Cytokine profile. A.** FCh from baseline (d21) of cytokine production detected in serum samples on day 28 post LMCV infection in mice receiving 3 doses of immune interventions. **B.** FCh from baseline (d21) of cytokine production detected in serum samples on day 34 post LMCV infection in mice receiving 5 doses of immune interventions.

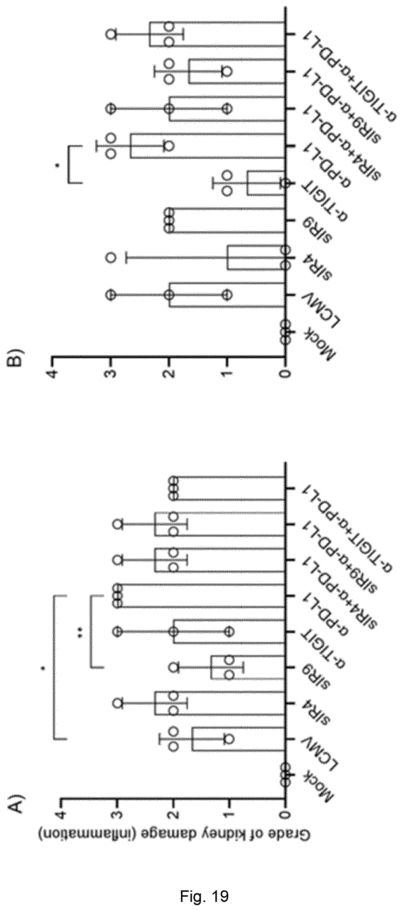

**Figure 19****:** Grade of kidney damage was determined as severity of chronic interstitial inflammation (ranking from 0 to 3) for each group of treatment after hematoxylin eosin staining of the kidneys (N=3/group) at two euthanasia times: 28 days post-infection, 3 doses of treatment (**A**) and 34 days post-infection, 5 doses of treatment (**B**).

### DETAILED DESCRIPTION OF THE INVENTION

### Composition of the invention

The authors of the present invention have found that compositions comprising a TIGIT variant, which contains part or of the extracellular region and an inhibitor of the PD-1/PD-L1 axis are capable of restoring the immune function of T-cells in patients suffering from T cell exhaustion and improving activation of dendritic cells, as well as inducing a pro-inflammatory hallmark in serum. Surprisingly, these compositions are comparatively more efficient in activating the immune system to revert immune exhaustion than similar compositions in which TIGIT inhibition is achieved by anti-TIGIT antibodies.

Accordingly, the invention relates to a composition comprising TIGIT variant which lacks the transmembrane and cytoplasmic domains and an inhibitor of the PD-1/PD-L1 axis. The composition may be used for treating or delaying an immune-related disease.

In a first aspect, the present invention relates to a composition, hereinafter "the composition of the invention" comprising:
a) a first component selected from the group consisting of:
   i) A polypeptide comprising a TIGIT variant that comprises the TIGIT Ig-like V-type domain and wherein said polypeptide lacks the TIGIT transmembrane and cytoplasmic domains and,
   ii) a polynucleotide encoding the polypeptide defined in i),
   and
b) a second component, which is an inhibitor of the PD-1/PD-L1 axis.

The term "comprising" or "comprises", as used herein, discloses also "consisting of" according to the generally accepted patent practice.

The terms "polypeptide" "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogues and amino acid mimetics that function in a manner similar to the naturally occurring amino acids. Furthermore, the term "amino acid" includes both D- and L-amino acids (stereoisomers).

The term "natural amino acids" or "naturally occurring amino acids" comprises the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine.

As used herein, the term "non-natural amino acid" or "synthetic amino acid" refers to a carboxylic acid, or a derivative thereof, substituted at position "a" with an amine group and being structurally related to a natural amino acid. Illustrative non- limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxy lysine, alio hydroxy lysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

The polypeptide of the present invention may also comprise non-amino acid moieties, such as for example, hydrophobic moieties (various linear, branched, cyclic, polycyclic or heterocyclic hydrocarbons and hydrocarbon derivatives) attached to the peptides; various protecting groups which are attached to the compound's terminals to decrease degradation. Suitable protecting functional groups are described in Green and Wuts, "Protecting Groups in Organic Synthesis", John Wiley and Sons, Chapters 5 and 7, 1991.

Chemical (non-amino acid) groups present in the polypeptide may be included in order to improve various physiological properties such as decreased degradation or clearance; decreased repulsion by various cellular pumps, improve various modes of administration, increased specificity, increased affinity, increased stability, bioavailability, solubility, decreased toxicity and the like.

"Mimetic" includes molecules that mimic the chemical structure of a peptidic structure and retain the functional properties of the peptidic structure. Approaches to designing peptide analogous, derivatives and mimetics are known in the art.

As used interchangeably herein, the terms "nucleic acid", "polynucleotide" and "nucleotide sequence" relate to any polymeric form of nucleotides of any length and composed of ribonucleotides or deoxyribonucleotides. The terms include both single-stranded and double-stranded polynucleotides, as well as modified polynucleotides (e.g., methylated, protected). Typically, the nucleic acid is a "coding sequence" which, as used herein, refers to a DNA sequence that is transcribed and translated into a polypeptide in a host cell when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A transcription termination sequence will usually be located 3' to the coding sequence.

The first component of the composition of the invention is selected from a group consisting of: a polypeptide comprising a TIGIT variant that comprises the TIGIT Ig-like V-type domain and wherein said polypeptide lacks the TIGIT transmembrane and cytoplasmic domains, and a polynucleotide encoding the previously defined polypeptide.

The term "TIGIT", as used herein, refers to the T cell immunoreceptor with Ig and ITIM domains), an inhibitory receptor expressed mainly in natural killer (NK), CD8+ T, CD4+ T and T regulatory (Treg) cells. The protein is defined in the NCBI database (release of 10 March 2024) with accession number NP_776160 for the human orthologue and with accession number NP_001139797 for the mouse orthologue.

The term "TIGIT variant" is to be understood as a polypeptide that comprises the TIGIT Ig-like V-type domain of a TIGIT polypeptide but that lacks the TIGIT transmembrane and cytoplasmic domains. The term includes any variant of the Ig-like V-type domain that results from the modification of one or more amino acid positions by deletion, insertion or substitution provided that the domain substantially maintains the ability to specifically bind CD155. In some embodiments, the TIGIT variant comprises the homodimerization domain, which facilitates the interaction of two TIGIT subunits in cis. In some embodiments, the TIGIT variant lacks the homodimerization domain, which facilitates the interaction of two TIGIT subunits in cis. In some embodiments, the TIGIT variant contains the three amino acidic sequences (VTQ, AX6G, and TYP) within the Ig-like V-type domain that allow the heterotetrameric interaction with CD155 in trans.

By way of illustration, variants of the polypeptide of the invention include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the polypeptide according to the invention and/or comprising the addition of 1 amino acid, 2 amino acids, 3 amino acids, 4 amino acids, 5 amino acids, 10 amino acids, 11 amino acids, 12 amino acids, 13 amino acids, 14 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxy terminus of the polypeptide according to the invention, and maintaining at least 50%, at least 60%, at least 70%, at least 75%, at least 80%, at least 85%, at least 90%, at least 91 %, at least 92%, at least 93%, at least 94%, at least 95%, at least 96%, at least 97%, at least 98%, at least 99%, or at least 100% of the activity of the sequence of the polypeptide according to the invention.

The variant of the polypeptide of the invention may also include post-translational modifications, such as glycosylation, acetylation, isoprenylation, myristoylation, proteolytic processing, etc.

In another embodiment, suitable variants of the polypeptide of the invention are those wherein one or more positions within the polypeptide of the invention contain an amino acid which is a conservative substitution of the amino acid present in the protein mentioned above. "Conservative amino acid substitutions" result from replacing one amino acid with another having similar structural and/or chemical properties. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example, by Dordo et al., (J. Mol. Biol, 1999, 217; 721-739) and Taylor et al., (J. Theor. Biol., 1986, 119:205-218).

TIGIT variants according to the present invention are those which substantially preserve the binding capability of native TIGIT to CD155 or which show increased affinity for CD155 binding with respect to native TIGIT. The binding capacity is usually measured by the binding affinity, which is a parameter that measures the strength of the sum total of non-covalent interactions between a single binding site of a molecule (e.g., a TIGT variant) and its binding partner (e.g.,CD155). Unless indicated otherwise, as used herein, "binding affinity" refers to intrinsic binding affinity which reflects a 1:1 interaction between members of a binding pair. The affinity of a molecule X for its partner Y can generally be represented by the dissociation constant (K_{D}). Affinity can be measured and/or expressed in a number of ways known in the art, including, but not limited to, equilibrium dissociation constant (K_{D}), and equilibrium association constant (K_{A}). The K_{D} is calculated from the quotient of k_{off}/kₒₙ, whereas K_{A} is calculated from the quotient of kₒₙ/k_{off}. kₒₙ refers to the association rate constant of, *e.g.*, an antibody or antigen-binding fragment thereof to an antigen, and k_{off} refers to the dissociation of the two members of the binding pair (e.g. the TIGIT variant and the CD155).

The affinity of the binding of the TIGIT variant to the CD155 may be measured by the dissociation constant or K_{D}. K_{D} values are measured by techniques known by the skilled in the art such as, for example ELISA, surface plasmon resonance (SPR), fluorescence anisotropy, Bio-Layer Interferometry, typically using OCTET(R) technology (Octet QKe system, ForteBio) or KinExA(R) (Kinetic Exclusion Assay) assay.

Considering that the binding affinity of native human TIGIT to human CD155 is of 1.3 nM, the suitable TIGIT variants for use according to the present invention include variants having a binding affinity of 10 nM or less, 9 nM or less, 8 nM or less, 7 nM or less, 6 nM or less, 5 nM or less, 4 nM or less. 3 nM or less, 2 nm or less, 1 nM or less or lower.

In a particular embodiment, the TIGIT variant essentially consists of amino acids 22 to 124 of the human TIGIT, wherein the numbering is as defined in the NCBI database entry (Release of 10 March 2024) under accession number NP_776160. In a more particular embodiment, the TIGIT variant is a polypeptide as defined in SEQ ID NO: 1 or SEQ ID NO: 39.

The term "essentially consisting", as used herein is used to define polypeptides that comprise the specifically defined region (amino acids 22 to 124 of the human TIGIT Ig-like V-type domain, wherein the numbering is as defined in the NCBI database entry NP_776160 and corresponding positions in other orthologues).

In some embodiments, the TIGIT variant contains amino acids 43 to 124 of human TIGIT as well as part or all of the region spanning from position 22 (the start of the mature TIGIT) to position 42 (the start of the Ig-like V-type domain) and/or part or all of the region spanning from position 125 (the end of the Ig-like V-type domain) to position 141 (the end of the extracellular region). Therefore, the TIGIT variant according to the invention may contain amino acids 22 to 124 of human TIGIT.

In an embodiment, the TIGIT variant essentially consists of amino acids 22 to 124 of human TIGIT. In an embodiment, the TIGIT variant essentially consists of amino acids 22 to 141 of human TIGIT. In an embodiment, the TIGIT variant essentially consists of amino acids 22 to 144 of human TIGIT.

In a particular embodiment, the TIGIT variant essentially consists of amino acids 20 to 119 of the mouse TIGIT, wherein the numbering is as defined in the NCBI database entry (Release of 24 May 2024) under accession number NP_001139797.

In a particular embodiment, the first component is a fusion protein comprising the TIGIT variant and a non-TIGIT polypeptide region. In another embodiment, the first component of the composition according to the invention is a polynucleotide encoding said fusion protein.

As used herein, the term "fusion protein" relates to proteins which consist of two or more functional domains derived from different proteins. A fusion protein may be obtained by conventional means, e.g., by means of gene expression of the nucleotide sequence encoding for said fusion protein in a suitable cell. The fusion protein, per definition, is never found in nature as such.

In some embodiments, wherein the first component of the composition according to the invention is a fusion protein comprising a non-TIGIT protein, i.e. a functional domain from a different protein, it is the functional domain of the fusion protein that confers the ability to form dimers, in which case the TIGIT variant contains the Ig-like V-type domain but does not contain the TIGIT homodimerization domain. Accordingly, in a preferred embodiment, the first component of the compositions according to the invention contains a fusion protein in which the TIGIT variant contains, essentially contains or consists of amino acids 41 to 122 of human TIGIT, wherein the numbering is as defined in the NCBI database entry (Release of 10 March 2024) under accession number NP_776160 or of amino acids 38 to 119 of the mouse TIGIT wherein the numbering is as defined in the NCBI database entry (Release of 24 May 2024) under accession number NP_001139797

In an embodiment, the non-TIGIT polypeptide region is a Fc monomer which is formed by the hinge region and the CH2 and CH3 domains of the heavy chain of an IgG heavy chain molecule.

The term "Fc monomer", as used herein refers to one of the two polypeptides that form the immunoglobulin Fc region and which comprise the comprising the hinge domain and the CH2/CH3 domains of the IgG heavy chain. The term "fragment crystallizable region" or "Fc region", as used herein, refers to the tail region of an antibody that interacts with cell surface receptors called Fc receptors and some proteins of the complement system.

The Fc is a dimer formed by two polypeptides, each one.

In a particular embodiment, when the first component of the composition according to the invention is a polypeptide comprising a TIGIT variant that comprises the TIGIT Ig-like V-type domain and wherein said polypeptide lacks the TIGIT transmembrane and cytoplasmic domains, the invention further relates to a homodimer that is formed by two monomers of the TIGIT variant. The homodimers are stabilized by the homodimerization domain in the TIGIT variant polypeptide which is found in amino acids 32 to 42 of human TIGIT wherein the wherein the numbering is as defined in the NCBI database entry (Release of 10 March 2024) under accession number NP_776160 or in amino acids 26 to 39 of murine TIGIT wherein the wherein the numbering is as defined in the NCBI database entry (Release of 10 March 2024) under accession number NP_001139797 .

In a particular embodiment, the first component of the composition according to the invention is a homodimer of two fusion proteins wherein each of the fusion protein comprises a TIGIT variant as defined above and an Fc monomer. In this case, the TIGIT variant that forms part of the fusion protein does not contain the homodimerization domains that appear in TIGIT.

The term "homodimer" refers to a structure comprising to identical monomers, for example, two identical monomer polypeptides.

The term the "homodimerization domain" refers to a domain of the human TIGIT Ig-like V-type domain or mouse TIGIT Ig-like V-type domain that facilities the interaction of two TIGIT subunits in cis. The homodimerization domain of the human TIGIT Ig-like V-type domain consists in: NISAEKGGSII (SEQ ID NO: 40). The homodimerization domain of the mouse TIGIT Ig-like V-type domain consists in: NISAEEGGSVI (SEQ ID NO: 41).

As used herein, the term "hinge", "hinge domain" or "antibody hinge region" refers to the domain of heavy chain constant region that joins the CH1 domain to the CH2 domain and includes the upper, middle, and lower portions of the hinge. The hinge provides varying levels of flexibility between the binding and effector regions of an antibody and also provides sites for intermolecular disulfide bonding between the two heavy chain constant regions.

The term "CH1 domain" refers to the heavy chain constant region linking the variable domain to the hinge in a heavy chain constant domain. The term "CH1 domain" includes wildtype CH1 domains as well as variants thereof (e.g., non-naturally-occurring CH1 domains or modified CH1 domains). For example, the term "CH1 domain" includes wildtype CH1 domains and variants having 1, 2, 3, 4, 5, 1-3, 1-5, 3-5 and/or at most 5, 4, 3, 2, or 1 mutations, e.g., substitutions, deletions or additions. Exemplary CH1 domains include CH1 domains with mutations that modify a biological activity of an antibody, such as ADCC, CDC or half-life.

The term "CH2 domain" refers to the heavy chain constant region linking the hinge to the CH3 domain in a heavy chain constant domain. The term "CH2 domain" includes wildtype CH2 domains, as well as variants thereof (e.g., non-naturally-occurring CH2 domains or modified CH2 domains). For example, the term "CH2 domain" includes wildtype CH2 domains and variants having 1, 2, 3, 4, 5, 1-3, 1-5, 3-5 and/or at most 5, 4, 3, 2, or 1 mutations, e.g., substitutions, deletions or additions. Exemplary CH2 domains include CH2 domains with mutations that modify a biological activity of an antibody, such as ADCC, CDC or half-life.

The term "CH3 domain" refers to the heavy chain constant region that is C-terminal to the CH2 domain in a heavy chain constant domain. The term "CH3 domain" includes wildtype CH3 domains, as well as variants thereof (e.g., non-naturally-occurring CH3 domains or modified CH3 domains). For example, the term "CH3 domain" includes wildtype CH3 domains and variants having 1, 2, 3, 4, 5, 1-3, 1-5, 3-5 and/or at most 5, 4, 3, 2, or 1 mutations, e.g., substitutions, deletions or additions. Exemplary CH3 domains include CH3 domains with mutations that modify a biological activity of an antibody, such as ADCC, CDC or half-life.

In a particular embodiment, the Fc monomer is selected from the group consisting of the human IgG1 Fc monomer, the human IgG1 Fc monomer carrying a S228P mutation, the mouse !gG2c Fc monomer, the mouse IgG2c Fc monomer carrying the ΔPCPP mutation and the mouse IgG1 Fc monomer.

In another embodiment, the Fc monomer is selected from the group consisting of SEQ ID NO: 3 (human IgG1 Fc monomer), SEQ ID NO: 4 (human S228P IgG1 Fc monomer), SEQ ID NO: 5 (mouse IgG2c Fc monomer), SEQ ID NO: 6 (mouse ΔPCPP IgG2c Fc monomer) or SEQ ID NO: 7 (mouse IgG1 Fc monomer). In some embodiments, the Fc monomer comprises a sequence at least 75%, at least 80%, at least 85%, at least 90%, at least 95%, at least 96%, at least 97%, at least 98%, at least 99% identical to SEQ ID NO: 3, SEQ ID NO: 4, SEQ ID NO: 5, SEQ ID NO: 6, SEQ ID NO: 7.

The terms "identical" or percent "identity" in the context of two or more nucleic acids or polypeptides refer to two or more sequences or subsequences that are the same or have a specified percentage of nucleotides or amino acid residues that are the same when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art which can be used to obtain alignments of amino acid or nucleotide sequences. Examples of algorithms suitable for determining sequence similarity include, but are not limited to, the BLAST, Gapped BLAST, and BLAST 2.0, WU-BLAST-2, ALIGN, and ALIGN-2 algorithms (Altschul S, et al., Nuc. Acids Res. 1977; 25:3389-3402, Altschul S, et al., J. Mol. Biol. 1990; 215:403-410, Altschul S, et al., Meth. Enzymol. 1996; 266:460-480, Karlin S, et al., Proc. Natl. Acad. Sci. USA 1990; 87:2264-2268, Karlin S, et al., Proc. Natl. Acad. Sci. USA 1993; 90:5873-5877, Genentech Corp, South San Francisco, CA, US, https://blast.ncbi.nlm.nih.gov/Blast.cgi, November 2021). Methods of alignment of sequences for comparison are well known in the art. Optimal alignment of sequences for comparison can be conducted, for instance, by the Smith-Waterman local homology algorithm, by the Needleman-Wunsch homology alignment algorithm, by the Pearson-Lipman similarity search method, by computerized implementations of these algorithms or by manual alignment and visual inspection (Smith T, et al., Adv. Appl. Math. 1981; 2:482-489, Needleman S, et al., J. Mol. Biol. 1970; 48:443-453, Pearson W, et al., Lipman D, Proc. Natl. Acad. Sci. USA 1988; 85:2444-2448, the GAP, BESTFIT, FASTA and TFASTA programs, Wisconsin Genetics Software Package, Genetics Computer Group, Madison, WI, USA; Ausubel F, et al., Eds., Short Protocols in Molecular Biology, 5th Ed. (John Wiley and Sons, Inc., New York, NY, USA, 2002)).

In a particular embodiment, the TIGIT variant is a fusion protein comprising the human TIGIT Ig-like V-type domain and a human IgG1 Fc monomer region. In a more particular embodiment the fusion protein consists of SEQ ID NO: 8.

In another particular embodiment, the TIGIT variant is a fusion protein comprising the human TIGIT Ig-like V-type domain and a human IgG4 Fc monomer region carrying a S228P mutation. In a more particular embodiment the fusion protein consists of SEQ ID NO: 9.

In a particular embodiment, the TIGIT variant is a fusion protein comprising the mouse TIGIT Ig-like V-type domain and a mouse IgG2c Fc monomer region. In a more particular embodiment the fusion protein consists of SEQ ID NO: 10.

In another particular embodiment, the TIGIT variant is a fusion protein comprising the mouse TIGIT Ig-like V-type domain and a mouse IgG2c Fc monomer region carrying a ΔPCPP mutation. In a more particular embodiment the fusion protein consists of SEQ ID NO: 11.

In a particular embodiment, the TIGIT variant is a fusion protein comprising the mouse TIGIT Ig-like V-type domain and a mouse IgG1 Fc monomer region. In a more particular embodiment the fusion protein consists of SEQ ID NO: 12.

In a another particular embodiment, the TIGIT variant is a fusion protein comprising the human TIGIT Ig-like V-type domain and a mouse IgG2c Fc monomer region. In a more particular embodiment the fusion protein consists of SEQ ID NO: 13.

In a particular embodiment, the TIGIT variant is a fusion protein comprising the human TIGIT Ig-like V-type domain and a mouse IgG2c Fc monomer region carrying a ΔPCPP mutation. In a more particular embodiment the fusion protein consists of SEQ ID NO: 14.

In a particular embodiment, the TIGIT variant is a fusion protein comprising the human TIGIT Ig-like V-type domain and a mouse IgG1 Fc monomer region. In a more particular embodiment the fusion protein consists of SEQ ID NO: 15.

In a particular embodiment, the first component is a polynucleotide selected from the group consisting of SEQ ID NO: 16 to 25.

In another embodiment, if the first component is a polynucleotide, then the polynucleotide further comprises a region that encodes a signal sequence which is fused in frame to the polypeptide that comprises the TIGIT variant.

As it is used herein, the term "signal sequence" or "signal peptide" refers to a peptide of a relatively short length, generally between 5 and 30 amino acid residues, directing proteins synthesized in the cell towards the secretory pathway. The signal peptide usually contains a series of hydrophobic amino acids adopting a secondary alpha helix structure. Additionally, many peptides include a series of positively-charged amino acids that can contribute to the protein adopting the suitable topology for its translocation. The signal peptide tends to have at its carboxyl end a motif for recognition by a peptidase, which is capable of hydrolyzing the signal peptide giving rise to a free signal peptide and a mature protein. The signal peptide can be cleaved once the protein of interest has reached the appropriate location. Any signal peptide may be used in the present invention. In a preferred embodiment, the signal sequence is the TIGIT native signal sequence, the azurocidin signal sequence or the CD5 signal sequence. In a more preferred embodiment, the signal sequence is as defined in SEQ ID NO: 26 (TIGIT native signal sequence), SEQ ID NO: 27 (CD5 signal sequence) or SEQ ID NO: 28 (azurocidin signal sequence).

In another embodiment, the composition of the invention wherein the polynucleotide is selected from the group consisting of SEQ ID NO: 29 to 38.

The second component of the composition of the invention consists of an inhibitor of the PD-1/PD-L1 axis.

The term "inhibitor of the PD-1/PD-L1 axis", as used herein, refers to a molecule that inhibits the interaction of a PD-L1 axis binding partner with either one or more of its binding partner, so as to remove T-cell dysfunction resulting from signaling on the PD-1 signaling axis - with a result being to restore or enhance T-cell function. As used herein, a PD-L1 axis binding antagonist includes a PD-L1 binding antagonist and a PD-1 binding antagonist as well as molecules that interfere with the interaction between PD-L1 and PD-1 (e.g., PD-L2-Fc fusion).

The term "PD-L1 binding antagonists" is a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative signal mediated by or through cell surface proteins expressed on T lymphocytes, and other cells, mediated signaling through PD-L1 or PD-1 so as render a dysfunctional T-cell less non-dysfunctional. In some embodiments of the method, the PD-1/PD-L1 signaling inhibitor is an anti-PD-L1 antibody or an antigen binding fragment thereof. In some embodiments of the method, the anti-PD-L1 antibody or the antigen binding fragment thereof is selected from the group consisting of polyclonal antibody, monoclonal antibody, Fab, scFv, diabody, triabody, minibody, VHH and sdAb.

In some embodiments of the method, the PD-L1 binding antagonist is an anti-PD-L1 antibody or the antigen binding fragment thereof is selected from the group consisting ofmanelimab, atezolizumab, avelumab, cosibelimab, durvalumab, envafolimab, socazolimab, BGB-A333, CK-301, CS-1001, FAZ-053, APL-502, MDX-1105, IMC-001, KD-005, Gensci-047, LY-3300054, SHR-1316, MSB-2311, AVA-004, CBT-502, JS-003, B12 and KY-1003. In some embodiments of the method, the anti-PD-L1 antibody or the antigen binding fragment thereof is B12.

In some embodiments of the method, the PD-L2 binding antagonist is an anti-PD-L2 antibody or an antigen binding fragment thereof. In a preferred embodiment, the anti-PD-L2 antibody is the mAb produced by clone 366C.9E5 (Merck catalog MABC1120).

The term "PD-1 binding antagonist" is a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes, and other cells, mediated signaling through PD-1 or PD-L1 so as render a dysfunctional T-cell less non-dysfunctional. In some embodiments of the method, the PD-1 binding antagonist is an anti-PD-1 antibody or the antigen binding fragment thereof is selected from the group consisting ofpidilizumab, cemiplimab, sintilimab, cetrelimab, spartalizumab, camrelizumab, tislelizumab, balstilimab, toripalimab, dostarlimab, ABBV-181, penpulimab, pembrolizumab, genolimzumab, retifanlimab, sasanlimab, AMP-224, AB122, F-520, MEDI-3387, MEDI-5771, MEDI-0680, SG-001, nivolumab, BCD-100, BAT-1306, BI-754091, CBT-501, GLS-010, LZM-009, Sym-021, CS-1003, HLX-10, AK-103, AM-0001, ENUM-244C8, ENUM-388D4, JTX-4014, RXI-762, STI-A1110, HLX-20, SSI-361, APL-501, TJ0141H, and SNA-01. In some embodiments of the method, the anti-PD-1 antibody or the antigen binding fragment thereof is toripalimab.

The terms "anti-PD1 antibody" or "anti-PD-1 neutralizing antibody" refer to an antibody that specifically binds to cell death protein 1 (PD-1) and inhibits the binding of PD-1 to its ligand PD-L1 and optionally inhibits the binding of PD-1 to its ligands PD-L1 and PD-L2. The anti-PD1 antibody thereby abolishes the suppressive effect of the PD-1/PD-L1 and/or PD-1/PD-L2 interaction on T cells.

The terms "anti-PD-L1 antibody" or "anti-PD-L1 neutralizing antibody" refer to an antibody that specifically binds to cell death protein 1 ligand (PD-L1) and inhibits the binding of PD-L1 to PD-1. The anti-PD-L1 antibody thereby abolishes the suppressive effect of the PD-L1/PD-1 interaction on T cells.

In another embodiment, the composition of the invention wherein the first component is the polypeptide as defined in SEQ ID NO: 14 and the second component is an anti-PD-L1 antibody or wherein the first component is the polypeptide as defined in SEQ ID NO: 15 and the second component is an anti-PD-L1 antibody. The anti-PD-L1 antibody used is an InVivoMAb anti-mouse PD-L1 (B7-H1) with a catalog number of BE0101 and a clone number of 10F.9G2^{™}.

In a more particular embodiment, the first component and the second component of the composition of the invention wherein the first component is a polypeptide are present at a ratio of from about 10,000:1 to about 1:10,000.

For example, the ratio of first component to second component may be from about 1:1 to about 1:500. In other embodiments, the ratio of first component to second component may be from about 1:1 to about 1:100, from about 1:1 to about 1:50, from about 1:10 to about 1:10,000, from about 1:10 to about 1:1,000, from about 1:10 to about 1:100, from about 1:10 to about 1:50, from about 1:30 to about 1:10,000, from about 1:30 to about 1:1,000, from about 1:30 to about 1:100, from bout 1:30 to about 1:50, from about 1:50 to about 1:10,000, from about 1:50 to about 1:1,000, from about 1:50 to about 1:100 or of about 1:50 or of about 1:10.

In another particular embodiment, the ratio of first component: to the second component is from about 1:1 to about 500:1, from about 1:1 to about 100:1, from about 1:1 to about 50:1, from about 10:1 to about 10,000:1, from about 10:1 to about 1,000:1, from about 10:1 to about 100:1, from about 10:1 to about 50:1. In particular embodiments, the ratio of first component: second component may be from about 30:1 to about 10,000:1, from about 30:1 to about 1,000:1, from about 30:1 to about 100:1, from about 30:1 to about 50:1, from about 50:1 to about 10,000:1, from about 50:1 to about 1,000:1, from about 50:1 to about 100:1. In a preferred embodiment, the ratio of first component to second component is of about 50:1. In another preferred embodiment the ratio of first component: second component is of about 10:1.

### Pharmaceutical composition of the invention

In as second aspect, the invention relates to a pharmaceutical composition, hereinafter the pharmaceutical composition of the invention, comprising a pharmaceutically effective amount of the composition of the invention and a pharmaceutically acceptable excipient.

As used herein, the term "pharmaceutical composition" refers to a form that allows the biological activity of the active ingredient contained to be effective and has an acceptable toxicity for the subject to which the composition is administered.

The term "pharmaceutically effective amount", as used herein, relates to the sufficient amount of a compound (i.e. of the combination of the invention) to provide the desired effect and it will generally be determined, by among other causes, the characteristics of the compound itself and the therapeutic effect to be achieved. It will also depend on the subject to be treated, the severity of the disease suffered by said subject, the chosen dosage form, administration route, etc.

The term "excipient" refers to a substance that aids the absorption of any of the components or compounds of the pharmaceutical composition of the invention, or stabilises the components or compounds and/or aids the preparation of the pharmaceutical composition in the sense of giving it consistency or flavours to make it more palatable. Thus, excipients may have the function, by way of example, but not limited to, binding the components (e.g. starches, sugars or cellulose), sweetening, colouring, protecting the active substance (e.g. to insulate it from air and/or moisture), filling a pill, capsule or any other presentation or a disintegrating function to facilitate the dissolution of the components, not excluding other excipients not listed in this paragraph. The term 'excipient' is therefore defined as a material which, included in the dosage forms, is added to the active substances or their associations to enable their preparation and stability, to modify their organoleptic properties or to determine the physical and chemical properties of the pharmaceutical composition and their bioavailability.

The expression "pharmaceutically acceptable excipient", as used herein, includes any and all solvents, dispersion media, coatings, antibacterial and antifungal agents, isotonic and absorption delaying agents that are physiologically compatible with the composition of the invention.

The "dosage form" is the configuration to which the active ingredients and excipients are adapted to provide a pharmaceutical composition or medicinal product. It is defined by the combination of the form in which the pharmaceutical composition is presented by the manufacturer and the form in which it is administered.

All the terms and embodiments previously describes are equally applicable to this aspect of the invention.

### Medical uses of the compositions of the invention in the treatment of immune-related disease

In a third aspect, the present invention relates to the composition of the invention or the pharmaceutical composition of the invention for use in medicine, hereinafter first medical use of the invention. Alternatively, the invention relates to the use of the composition of the invention or the pharmaceutical composition of the invention for the manufacture of a medicament.

In a fourth aspect, the present invention relates to the composition of the invention or the pharmaceutical composition of the invention for use in a method for treating or delaying an immune-related disease, hereinafter second medical use of the invention. Alternatively, the invention relates to the use of the composition of the invention or the pharmaceutical composition of the invention for the manufacture of a medicament for treating or delaying an immune-related disease. Alternatively, the invention relates to a method for treating or delaying an immune-related disease, the method comprising administering to the subject an effective amount of the composition of the invention or the pharmaceutical composition of the invention.

The terms "treatment", "treat" or "treating" as used herein refer to the administration of the composition of the invention or the pharmaceutical composition for controlling the progression of a disease after its clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival compared with the expected survival if treatment was not applied. Within the context of the present invention, the terms "treat" and "treatment" refer specifically to stopping or slowing the infection and destruction of healthy CD4+ T cells by Human Immunodeficiency Virus (HIV) together with the immune exhaustion derived from chronic HIV, chronic hepatitis, Hepatitis C Virus (HCV), retrovirus, Herpes Simplex virus, such Cytomegalovirus (CMV), Epstein Bar Virus (EBV), Varicella Zoster Virus (VZV) and Tuberculosis infected subject. It also refers to the stopping and slowing of the onset of symptoms of the acquired immunodeficiency disease (AIDS), such as extremely low CD4+ T cell count and repeated infections by opportunistic pathogens. Beneficial or desired clinical results include, but are not limited to, an increase in absolute naive CD4+ T cell count (range 10-3520), an increase in the percentage of CD4+ T cell over total circulating immune cells (range 1-50%), or an increase in CD4+ T cell count as a percentage of normal CD4+ T cell count in an uninfected subject (range 1-161%). "Treatment" can also mean prolonging survival of the infected subject as compared to expected survival if the subject does not receive any chronic hepatitis, Hepatitis C Virus (HCV), retrovirus, Human Immunodeficiency Virus (HIV), Herpes Simplex virus, such Cytomegalovirus (CMV), Epstein Bar Virus (EBV), Varicella Zoster Virus (VZV) and/or Tuberculosis targeted treatment.

The terms "delay" or "delaying", as used herein refers to a cessation or slowing in the decline of one or more parameters of the immune-related disease in a subject or a maintenance of a level of one or more parameters of the immune-related disease.

The term "subject", as used herein, refers to an individual, plant or animal, such as a human, a nonhuman primate (e.g., chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term does not denote a particular age or sex. In a preferred embodiment of the invention, the subject is a human.

As used herein, the term "immune related disease" relates to a disease in which the immune system is involved in the pathogenesis of the disease, or in which cell proliferation occurs. Examples of immune-related diseases object of this invention are inflammatory diseases, in particular autoimmune diseases, and cancers.

In another embodiment, the immune related disease is a chronic viral infection, a chronic bacterial infection, and a tumor.

As used herein, the term "chronic viral infection" refers to a subject afflicted or infected with a chronic virus. In one embodiment, the chronic viral infection is selected, but not limited, from the group consisting of: HCV, HIV, CMV, EBV and VZV.

The term "HCV" or "Hepatitis C virus", as used herein, refers to a single-stranded, positive-sense RNA virus member of the genus Hepacivirus in the family Flaviviridae (Rosen H, et al., NEJM 2011; 364(25):2429-2438). HCV may lead to liver disease and cirrhosis. In some cases, those with cirrhosis may develop serious complications such as liver failure, liver cancer, or dilated blood vessels in the esophagus and stomach.

The term "HIV", as used herein, include HIV-1 and HIV-2, SHIV and SIV. "HIV-1" means the human immunodeficiency virus type-1. HIV-1 includes, but is not limited to, extracellular virus particles and the forms of HIV-1 associated with HIV-1 infected cells. The HIV-1 virus may represent any of the known major subtypes (Classes A, B, C, D E, F, G and H) or outlying subtype (Group O) including laboratory strains and primary isolates. "HIV-2" means the human immunodeficiency virus type-2. HIV-2 includes, but is not limited to, extracellular virus particles and the forms of HIV-2 associated with HIV-2 infected cells. The term "SIV" refers to simian immunodeficiency virus which is an HIV-like virus that infects monkeys, chimpanzees, and other nonhuman primates. SIV includes, but is not limited to, extracellular virus particles and the forms of SIV associated with SIV infected cells.

The term "HIV exposure", as used herein, refers to the contact of an uninfected subject with a subject having an HIV infection or AIDS, or the contact with body fluids from such HIV-infected subject, in which such fluids from the infected subject contact a mucous membrane, a cut or abrasion in the tissue (e.g., needle stick, unprotected sexual intercourse), or other surface of the uninfected subject in such a way that the virus could be transmitted from the infected subject or infected subject's body fluids to the uninfected subject.

The term "HIV infection", as used herein, refers to indications of the presence of the HIV virus in an individual including asymptomatic seropositivity, AIDS-related complex (ARC), and acquired immunodeficiency syndrome (AIDS).

The terms "CMV" or "Cytomegalovirus", as used herein, refers to a genus of viruses in the order Herpesvirales, family Herpesviridae, subfamily Betaherpesvirinae. The CMV genus comprises 11 species including human betaherpesvirus 5 (HCMV, human cytomegalovirus, HHV-5), which is the species that infects humans. Diseases associated with HHV-5 include mononucleosis and pneumonia (ENA accession number GU980198).

The term "CMV therapy", as used herein, refers to any therapies, including blood products, immune therapies and drug therapies, approved or currently under evaluation for the prevention, inhibition of the progression or treatment of CMV or its related diseases. Examples of CMV therapies include antivirals such as Ganciclovir (CAS [82410-32-0]).

The term "EBV" or "Epstein-Barr virus", as used herein, refers to a double-stranded DNA virus member of the herpes virus family (Zanella M, et al., Clinical Microbiol Rev 2020; 33(4):e00027-20.0). EBV spreads most commonly through bodily fluids, primarily saliva. EBV can cause infectious mononucleosis, also called mono, and other illnesses.

The term "VZV" or "Varicella-Zoster virus", as used herein, refers to a human herpesvirus 3 (HHV-3, HHV3) or human alphaherpesvirus 3 (taxonomically), is one of nine known herpes viruses that can infect humans. It causes chickenpox (varicella) commonly affecting children and young adults, and shingles (herpes zoster) in adults but rarely in children. VZV infections are species-specific to humans.

The term "VZV infection", as used herein, refers to indications of the presence of the VZV in an individual.

The term "chronic bacterial infection" is a bacterial infection that is of a long duration or frequent recurrence. For example, a chronic middle ear infection, or otitis media, can occur when the Eustachian tube becomes blocked repeatedly due to allergies, multiple infections, ear trauma, or swelling of the adenoids. The definition of "long duration" will depend upon the particular infection. For example, in the case of a chronic middle ear infection, it may last for weeks to months. Other known chronic bacterial infections include urinary tract infection (most commonly caused by *Escherichia coli* and/or *Staphylococcus saprophyticus*), gastritis (most commonly caused by *Helicobacter pylori*), respiratory infection (such as those commonly afflicting patents with cystic fibrosis, most commonly caused by *Pseudomonas aeruginosa*), pyelonephritis (most commonly caused by *Proteus* species, *Escherichia coli* and/or *Pseudomonas* species), osteomyelitis (most commonly caused by *Staphylococcus aureus*, but also by *Escherichia coli*), bacteremia, skin infection, rosacea, acne, chronic wound infection, infectious kidney stones (can be caused by *Proteus mirabilis*), bacterial endocarditis, and sinus infection.

As used herein, the term "cancer" or "tumor" or "tumor disease" refers to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. As used herein, the term cancer includes, but is not limited to breast cancer, ovarian cancer, pancreatic cancer, prostate cancer, lung cancer, colorectal cancer, stomach/gastric cancer, endometrial/uterine/cervical cancer, bladder cancer, head and neck cancer, leukemia, cancer of the heart, of the small intestine, spleen, kidney, brain, skin, bone, bone marrow, blood, thymus, womb, testicles, hepatobiliary system and liver, sarcoma, cholangiocarcinoma, glioblastoma, multiple myeloma, lymphoma, adenoma, angiosarcoma, astrocytoma, epithelial carcinoma, germinoma, glioma, hemangioendothelioma, hemangiosarcoma, hematoma, hepatoblastoma, medulloblastoma, melanoma, neuroblastoma, hepatobiliary cancer, osteosarcoma, retinoblastoma, rhabdomyosarcoma, sarcoma and teratoma. Furthermore, this term includes acrolentiginous melanoma, actinic keratosis adenocarcinoma, adenoid cystic carcinoma, adenomas, adenosarcoma, adenosquamus carcinoma, astrocytic tumors, Bartholin gland carcinoma, basal cell carcinoma, bronchial gland carcinoma, capillary carcinoid, carcinoma, carcinosarcoma, cystadenoma, endodermal sinus tumor, endometrial hyperplasia, endometrial stromal sarcoma, endometrioid adenocarcinoma, ependymal sarcoma, Ewing sarcoma, focal nodular hyperplasia, germ cell tumors, glucagonoma, hemangioblastoma, hemagioendothelioma, hemagioma, hepatic adenoma, hepatic adenomastosis, hepatocellular carcinoma, hepatobiliary cancer, insulinoma, intraepithelial neoplasia, squamous cell intraepithelial neoplasia, invasive squamous-cell carcinoma, large cell carcinoma, leiomyosarcoma, melanoma, malignant melonoma, malignant mesothelial tumor, medulloblastoma, medulloepithelioma, mucoepidermoid carcinoma, neuroblastoma, neuroepithelial adenocarcinoma, nodular melanoma, osteosarcoma, papillary serous adenocarcinoma, pituitary tumors, plasmacytoma, pseudosarcoma, pulmonary blastoma, renal cell carcinoma, retinoblastoma, rhabdomyosarcoma, serous carcinoma, microcytic carcinoma, soft tissue carcinoma, somatostatin secreting tumor, squamous carcinoma, squamous cell carcinoma, undifferentiated carcinoma, uveal melanoma, verrucous carcinoma, vipoma, Wilm tumor, intracerebral cancer, rectal cancer, astrocytoma, microcytic cancer and non-microcytic cancer, metastatic melanoma, androgen-independent metastatic prostate cancer, androgen-dependent metastatic prostate cancer. The term cancer includes both primary tumors as well as metastatic tumors.

In a more particular embodiment, the first component and the second component of the composition of the invention are administered simultaneously or sequentially.

All previous definitions and embodiments related to previous aspects are also applicable to the third and fourth aspects and their embodiments.

### Methods for reducing immune toxicity in a patient that is being treated with an inhibitor of the PD-1/PD-L1 axis using soluble TIGIT variants

In a fifth aspect, the present invention relates to a polypeptide comprising a TIGIT variant that comprises the TIGIT Ig-like V-type domain and wherein said polypeptide lacks the TIGIT transmembrane and cytoplasmic domains or a polynucleotide encoding said polypeptide for use in a method for reducing immune toxicity in a patient that is being treated with an inhibitor of the PD-1/PD-L1 axis, hereinafter second medical use of the invention.

The term "inhibitor of the PD-1/PD-L1 axis", as used herein, refers to a molecule that inhibits the interaction of a PD-L1 axis binding partner with either one or more of its binding partner, so as to remove T-cell dysfunction resulting from signaling on the PD-1 signaling axis - with a result being to restore or enhance T-cell function. As used herein, a PD-L1 axis binding antagonist includes a PD-L1 binding antagonist and a PD-1 binding antagonist as well as molecules that interfere with the interaction between PD-L1 and PD-1 (e.g., PD-L2-Fc fusion).

The term "PD-L1 binding antagonists" is a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative signal mediated by or through cell surface proteins expressed on T lymphocytes, and other cells, mediated signaling through PD-L1 or PD-1 so as render a dysfunctional T-cell less non-dysfunctional. In some embodiments of the method, the PD-1/PD-L1 signaling inhibitor is an anti-PD-L1 antibodyor an antigen binding fragment thereof. In some embodiments of the method, the anti-PD-L1 antibody or the antigen binding fragment thereof is selected from the group consisting of polyclonal antibody, monoclonal antibody, Fab, scFv, diabody, triabody, minibody, VHH and sdAb.

In some embodiments of the method, the PD-L1 binding antagonist is an anti-PD-L1 antibody or the antigen binding fragment thereof is selected from the group consisting ofmanelimab, atezolizumab, avelumab, cosibelimab, durvalumab, envafolimab, socazolimab, BGB-A333, CK-301, CS-1001, FAZ-053, APL-502, MDX-1105, IMC-001, KD-005, Gensci-047, LY-3300054, SHR-1316, MSB-2311, AVA-004, CBT-502, JS-003, B12 and KY-1003. In some embodiments of the method, the anti-PD-L1 antibody or the antigen binding fragment thereof is B12.

The term "PD-1 binding antagonist" is a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes, and other cells, mediated signaling through PD-1 or PD-L1 so as render a dysfunctional T-cell less non-dysfunctional. In some embodiments of the method, the PD-1 binding antagonist is an anti-PD-1 antibody or the antigen binding fragment thereof is selected from the group consisting ofpidilizumab, cemiplimab, sintilimab, cetrelimab, spartalizumab, camrelizumab, tislelizumab, balstilimab, toripalimab, dostarlimab, ABBV-181, penpulimab, pembrolizumab, genolimzumab, retifanlimab, sasanlimab, AMP-224, AB122, F-520, MEDI-3387, MEDI-5771, MEDI-0680, SG-001, nivolumab, BCD-100, BAT-1306, BI-754091, CBT-501, GLS-010, LZM-009, Sym-021, CS-1003, HLX-10, AK-103, AM-0001, ENUM-244C8, ENUM-388D4, JTX-4014, RXI-762, STI-A1110, HLX-20, SSI-361, APL-501, TJ0141H, and SNA-01. In some embodiments of the method, the anti-PD-1 antibody or the antigen binding fragment thereof is toripalimab.

In a particular embodiment, the polypeptide or polynucleotide for use according to the fifth aspect of the invention contains a TIGIT variant that contains both the homodimerization domain and the Ig-like V-type domain. Preferably, the TIGIT variant comprises, consists or essentially consists of amino acids 22 to 124 of human TIGIT wherein the numbering is as defined in the NCBI database entry (Release of 10 March 2024) under accession number NP_776160 or amino acids 20 to 119 of the mouse TIGIT wherein the numbering is as defined in the NCBI database entry (Release of 24 May 2024) under accession number NP_001139797. In another more particular embodiment, the TIGIT variant is a polypeptide as defined in SEQ ID NO: 1 or SEQ ID NO: 39.

In a preferred embodiment, the polypeptide or polynucleotide for use according to the fifth aspect of the invention, which is a fusion protein comprising the TIGIT variant and a non-TIGIT polypeptide region or a polynucleotide encoding said fusion protein.

In a particular embodiment, the non-TIGIT polypeptide region is a Fc monomer region which is formed by the hinge region and the CH2 and CH3 domains of an IgG molecule.

In another embodiment, the Fc monomer region is selected from the group consisting of the human IgG1 Fc monomer, the human IgG1 Fc monomer carrying a S228P mutation, the mouse !gG2c Fc monomer, the mouse IgG2c Fc monomer carrying the ΔPCPP mutation and the mouse IgG1 Fc monomer.

In a more particular embodiment, the Fc monomer region is selected from the group consisting of SEQ ID NO: 3 (human IgG1 Fc monomer), SEQ ID NO: 4 (human IgG1 Fc monomer carrying a S228P mutation), SEQ ID NO: 5 (mouse IgG2c Fc monomer), SEQ ID NO: 6 (mouse IgG2c Fc monomer carrying the ΔPCPP mutation) or SEQ ID NO: 7 (mouse IgG1 Fc monomer).

In particular embodiment, the TIGIT variant is a fusion protein comprising the human TIGIT Ig-like V-type domain and a human IgG1 Fc monomer region. In a more particular embodiment the fusion protein consists of SEQ ID NO: 8.

In particular embodiment, the TIGIT variant is a fusion protein comprising the human TIGIT Ig-like V-type domain and a human IgG4 Fc monomer region carrying a S228P mutation. In a more particular embodiment the fusion protein consists of SEQ ID NO: 9.

In particular embodiment, the TIGIT variant is a fusion protein comprising the mouse TIGIT Ig-like V-type domain and a mouse !gG2c Fc monomer region. In a more particular embodiment the fusion protein consists of SEQ ID NO: 10.

In particular embodiment, the TIGIT variant is a fusion protein comprising the mouse TIGIT Ig-like V-type domain and a mouse !gG2c Fc monomer region carrying a ΔPCPP mutation. In a more particular embodiment the fusion protein consists of SEQ ID NO: 11.

In particular embodiment, the TIGIT variant is a fusion protein comprising the mouse TIGIT Ig-like V-type domain and a mouse IgG1 Fc monomer region. In a more particular embodiment the fusion protein consists of SEQ ID NO: 12.

In particular embodiment, the TIGIT variant is a fusion protein comprising the human TIGIT Ig-like V-type domain and a mouse !gG2c Fc monomer region. In a more particular embodiment the fusion protein consists of SEQ ID NO: 13.

In particular embodiment, the TIGIT variant is a fusion protein comprising the human TIGIT Ig-like V-type domain and a mouse !gG2c Fc monomer region carrying a ΔPCPP mutation. In a more particular embodiment the fusion protein consists of SEQ ID NO: 14.

In particular embodiment, the TIGIT variant is a fusion protein comprising the human TIGIT Ig-like V-type domain and a mouse IgG1 Fc monomer region. In a more particular embodiment the fusion protein consists of SEQ ID NO: 15.

In another embodiment, the polypeptide or polynucleotide for use according to the fifth aspect of the invention which is selected from the group consisting of SEQ ID NO: 16 to 25.

In a more particular embodiment, the polypeptide or polynucleotide for use according to the fifth aspect of the invention wherein, if the first component is a polynucleotide, then the polynucleotide further comprises a region that encodes a signal sequence which is fused in frame to the polypeptide that comprises the TIGIT variant.

In a preferred embodiment, the polypeptide or polynucleotide for use according to the fifth aspect of the invention wherein the signal sequence is as defined in SEQ ID NO: 26 (TIGIT native signal sequence), SEQ ID NO: 27 (CD5 signal sequence) or SEQ ID NO: 28 (azurocidin signal sequence).

In a particular embodiment, the polypeptide or polynucleotide for use according to the fifth aspect of the invention wherein the polynucleotide is selected from the group consisting of SEQ ID NO: 29 to 38.

All the terms and embodiments previously describes are equally applicable to this aspect of the invention.

### Further aspects of the invention

1. A composition comprising:
   a) a first component selected from the group consisting of:
      i) a polypeptide comprising a TIGIT variant that comprises the TIGIT Ig-like V-type domain and wherein said polypeptide lacks the TIGIT transmembrane and cytoplasmic domains and,
      ii) a polynucleotide encoding the polypeptide defined in i),
      and
   b) a second component, which is an inhibitor of the PD-1/PD-L1 axis.
2. The composition according to aspect 1 wherein the TIGIT variant essentially consists of amino acids 22 to 124 of human TIGIT, wherein the numbering is as defined in the NCBI database entry (Release of 10 March 2024) under accession number NP_776160 or of amino acids 20 to 119 of the mouse TIGIT wherein the numbering is as defined in the NCBI database entry (Release of 24 May 2024) under accession number NP_001139797.
3. The composition according to aspect 2 wherein the TIGIT variant is a polypeptide as defined in SEQ ID NO: 1 or SEQ ID NO: 39 or SEQ ID NO: 2.
4. The composition according to aspect 1 wherein the first component is a fusion protein comprising the TIGIT variant and a non-TIGIT polypeptide region or a polynucleotide encoding said fusion protein.
5. The composition according to aspect 4 wherein the TIGIT variant essentially consists of amino acids 41 to 122 of human TIGIT, wherein the numbering is as defined in the NCBI database entry (Release of 10 March 2024) under accession number NP_776160 or of amino acids 38 to 119 of the mouse TIGIT wherein the numbering is as defined in the NCBI database entry (Release of 24 May 2024) under accession number NP_001139797.
6. The composition according to aspects 4 or 5 wherein the non-TIGIT polypeptide region is a Fc monomer which is formed by the hinge region and the CH2 and CH3 domains of an IgG molecule.
7. The composition according to aspect 6 wherein the Fc monomer is selected from the group consisting of the human IgG1 Fc monomer, the human IgG1 Fc monomer carrying a S228P mutation, the mouse !gG2c Fc monomer, the mouse IgG2c Fc monomer carrying the ΔPCPP mutation and the mouse IgG1 Fc monomer.
8. The composition according to aspect 7 wherein the Fc monomer is selected from the group consisting of SEQ ID NO: 3, 4, 5, 6 or 7.
9. The composition according to aspect 8 wherein the TIGIT variant is selected from the group consisting of:
   a) A fusion protein comprising the human TIGIT Ig-like V-type domain and a human IgG1 Fc monomer,
   b) A fusion protein comprising the human TIGIT Ig-like V-type domain and a human IgG4 Fc monomer carrying a S228P mutation,
   c) A fusion protein comprising the mouse TIGIT Ig-like V-type domain and a mouse !gG2c Fc monomer,
   d) A fusion protein comprising the mouse TIGIT Ig-like V-type domain and a mouse !gG2c Fc monomer carrying a ΔPCPP mutation,
   e) A fusion protein comprising the mouse TIGIT Ig-like V-type domain and a mouse IgG1 Fc monomer,
   f) A fusion protein comprising the human TIGIT Ig-like V-type domain and a mouse !gG2c Fc monomer,
   g) A fusion protein comprising the human TIGIT Ig-like V-type domain and a mouse !gG2c Fc monomer carrying a ΔPCPP mutation and
   h) A fusion protein comprising the human TIGIT Ig-like V-type domain and a mouse IgG1 Fc monomer.
10. The composition according to aspect 9 wherein the fusion protein is selected from the group consisting of SEQ ID NO: 8 to 15.
11. The composition according to any of aspects 1 to 4 wherein the first component is provided as a homodimer of polypeptides comprising the TIGIT variant or according to any of aspects 5 to 10 wherein the first component is provided as a homodimer of two fusion proteins.
12. The composition according to any of aspects 1 to 11 wherein the first component is a polynucleotide selected from the group consisting of SEQ ID NO: 16 to 25.
13. The composition according to any of aspects 1 to 12 wherein, if the first component is a polynucleotide, then the polynucleotide further comprises a region that encodes a signal sequence which is fused in frame to the polypeptide that comprises the TIGIT variant.
14. The composition according to aspect 13 wherein the signal sequence is as defined in SEQ ID NO: 26, 27 or 28.
15. The composition according to aspects 13 wherein the polynucleotide is selected from the group consisting of SEQ ID NO: 29 to 38.
16. The composition according to any of aspects 1 to 15, wherein the inhibitor of the PD-1/PD-L1 axis is selected from the group consisting of a PD-1 binding antagonist, a PD-L 1 binding antagonist and a PD-L2 binding antagonist.
17. The composition according to aspect 16 wherein the PD-1 binding antagonist is anti-PD-1 antibody or an antigen-binding fragment thereof, the PD-L 1 binding antagonist is an anti-PD-L1 antibody or an antigen-binding fragment thereof or the PD-L2 binding antagonist is an anti-PD-L2 antibody or an antigen-binding fragment thereof.
18. The composition according to aspect 17 wherein
   a) the PD-1 binding antagonist is selected from nivolumab, pembrolizumab, lambrolizumab pidilizumab, Cemiplimab, dostarlimab, toripalimab, tislelizumab camrelizumab, sintilimab and spartalizumab,
   b) the PD-L1 binding antagonist is selected from the group consisting of atezolizumab, durvalumab and avelumab, the BMS-936559 antibody, durvalumab and the 10F.9G 2 antibody,
   c) the PD-L2 binding antagonist is anti-PD-L2 mAb, preferably the mAb produced by clone 366C.9E5.
19. The composition according to aspect 18 wherein the first component is the polypeptide as defined in SEQ ID NO: 14 and the second component is an anti-PD-L1 antibody or wherein the first component is the polypeptide as defined in SEQ ID NO: 15 and the second component is an anti-PD-L1 antibody.
20. A pharmaceutical composition comprising a pharmaceutically effective amount of the composition according to any one of aspects 1 to 19 and a pharmaceutically acceptable excipient.
21. The composition according to any one of aspects 1 to 19 or the pharmaceutical composition according to aspect 20 for use in medicine.
22. The composition according to any one of aspects 1 to 19 or the pharmaceutical composition according to aspect 20 for use in a method for treating or delaying an immune-related disease.
23. The composition or pharmaceutical composition for use according to aspect 22 wherein the immune related disease is a chronic viral infection, a chronic bacterial infection, and a tumor.
24. The composition or pharmaceutical composition for use according to aspects 22 or 23 wherein the first component and the second component are administered simultaneously or sequentially.
25. A polypeptide comprising a TIGIT variant that comprises the TIGIT Ig-like V-type domain and wherein said polypeptide lacks the TIGIT transmembrane and cytoplasmic domains or a polynucleotide encoding said polypeptide for use in a method for reducing immune toxicity in a patient that is being treated with an inhibitor of the PD-1/PD-L-1 axis.
26. The polypeptide or polynucleotide for use according to aspect 25 wherein the TIGIT variant essentially consists of amino acids 22 to 124 of the human TIGIT wherein the numbering is as defined in the NCBI database entry (Release of 10 March 2024) under accession number NP_776160 or of amino acids 20 to 119 of the mouse TIGIT wherein the numbering is as defined in the NCBI database entry (Release of 24 May 2024) under accession number NP_001139797.
27. The polypeptide or polynucleotide for use according to aspect 26 wherein the TIGIT variant is a polypeptide as defined in SEQ ID NO: 1 or SEQ ID NO: 39 or SEQ ID NO: 2.
28. The polypeptide or polynucleotide for use according to aspect 25 which is a fusion protein comprising the TIGIT variant and a non-TIGIT polypeptide region or a polynucleotide encoding said fusion protein.
29. The polypeptide or polynucleotide for use according to aspect 26 wherein the TIGIT variant essentially consists of amino acids 41 to 122 of human TIGIT, wherein the numbering is as defined in the NCBI database entry (Release of 10 March 2024) under accession number NP_776160 or of amino acids 38 to 119 of the mouse TIGIT wherein the numbering is as defined in the NCBI database entry (Release of 24 May 2024) under accession number NP_001139797.
30. The polypeptide or polynucleotide for use according to aspects 28 or 29 wherein the non-TIGIT polypeptide region is a Fc monomer region which is formed by the hinge region and the CH2 and CH3 domains of an IgG molecule.
31. The polypeptide or polynucleotide for use according to aspect 30 wherein the Fc monomer region is selected from the group consisting of the human IgG1 Fc monomer, the human IgG1 Fc monomer carrying a S228P mutation, the mouse IgG2c Fc monomer, the mouse IgG2c Fc carrying the ΔPCPP mutation and the mouse IgG1 Fc monomer.
32. The polypeptide or polynucleotide for use according to aspect 31 wherein the Fc monomer region is selected from the group consisting of SEQ ID NO:3, 4, 5, 6 or 7.
33. The polypeptide or polynucleotide for use according to aspect 32 wherein the TIGIT variant is selected from the group consisting of:
   a) A fusion protein comprising the human TIGIT Ig-like V-type domain and a human IgG1 Fc monomer region,
   b) A fusion protein comprising the human TIGIT Ig-like V-type domain and a human IgG4 Fc monomer region carrying a S228P mutation,
   c) A fusion protein comprising the mouse TIGIT Ig-like V-type domain and a mouse !gG2c Fc monomer region,
   d) A fusion protein comprising the mouse TIGIT Ig-like V-type domain and a mouse !gG2c Fc monomer region carrying a ΔPCPP mutation,
   e) A fusion protein comprising the mouse TIGIT Ig-like V-type domain and a mouse IgG1 Fc monomer region,
   f) A fusion protein comprising the human TIGIT Ig-like V-type domain and a mouse !gG2c Fc monomer region,
   g) A fusion protein comprising the human TIGIT Ig-like V-type domain and a mouse !gG2c Fc monomer region carrying a ΔPCPP mutation and
   h) A fusion protein comprising the human TIGIT Ig-like V-type domain and a mouse IgG1 Fc monomer region.
34. The polypeptide or polynucleotide for use according to aspect 33 wherein the fusion protein is selected from the group consisting of SEQ ID NO: 8 to 15.
35. The polypeptide or polynucleotide for use according to any of aspects 25 to 34 which is selected from the group consisting of SEQ ID NO: 16 to 25.
36. The polypeptide or polynucleotide for use according to any of aspects 25 to 35 wherein, if the first component is a polynucleotide, then the polynucleotide further comprises a region that encodes a signal sequence which is fused in frame to the polypeptide that comprises the TIGIT variant.
37. The polypeptide or polynucleotide for use according to aspect 36 wherein the signal sequence is as defined in SEQ ID NO: 26, 27 or 28.
38. The polypeptide or polynucleotide for use according to aspect 37 wherein the polynucleotide is selected from the group consisting of SEQ ID NO: 29 to 38.

The invention is defined below by means of the following examples which are to be construed as purely illustrative and not limitative of the scope of the invention.

### EXAMPLES

### Materials

### In silico modelling by I-TASSER

The Iterative Threading ASSEmbly Refinement (I-TASSER) modelling tool was used to predict the 3D structures of the sIRs. The sIR amino acid sequences were submitted for in silico modelling, and 3D models were obtained. From the top 5 final models, the highest C-score model was selected for visualization and analysis using Pymol software. The secondary and tertiary structures were aligned to the X-ray diffraction protein structure of TIGIT (PDB - 3Q0H). Finally, predicted solvent accessibility was analysed to determine buried and highly exposed residues in the homodimerization domains and the VTQ, AX6G, and TYP sequences.

### Plasmids design and production

All DNA sequences encoding sIR proteins (Fig. 1-2) were synthesised using the Gene Synthesis service (Genscript). The DNA sequence was optimised for best codon usage and GC content in human cells using the Optimum^{™} Codon Tool. The final DNA sequence was cloned into pcDNA3.1/Hygro (+) or pcDNA3.4- TOPO vectors. The One Shot^{™} OmniMAX^{™} 2 T1R Chemically Competent E. coli cells (Ref. C854003, Invitrogen) were used following the manufacturer's instructions. Briefly, the plasmids encoding sIR were resuspended in nuclease- free H2O to obtain a stock concentration of 100 ng/µL. Then, the sIRs' plasmid DNA (100 ng) was added to the One-Shot competent E. *coli* cells, which were incubated on ice for 30 min. Then, cells were heat-shocked at 42°C for 30 seconds without shaking, followed by a 2-minute incubation on ice. To recover the culture, pre-warmed S.O.C. medium (250 µL) was added to each vial, and the cell culture was incubated at 37°C for 1 hour in a shaking incubator at 225 rpm. Finally, an aliquot of the recovered transformation culture (25-100 µL) was spread onto a pre-warmed selective LB agar plate containing 10 µg/mL ampicillin, followed by overnight incubation at 37°C and 225 rpm. The next day, the colonies were selected from the plate and grown in 3mL of ampicillin-LB selective media at 37°C for 4 h in a shaking incubator at 225 rpm. After that, the volume was escalated to 200 mL, and the cultures were grown overnight at 37°C in a shaking incubator at 225 rpm for plasmid DNA purification. Then, cell cultures were pelleted by centrifugation for 30 min at 3000 g to obtain cell pellets. According to the manufacturer's instructions, plasmid DNA was purified with ZymoPURE^{™} II Plasmid Maxiprep Kit (Zymo Research) and DNA was sterilised with 0,22 µm filter. DNA concentration was determined by measuring absorbance at 260 nm using a NanoDrop One/One (ThermoFisher Scientific). Plasmid digestion was analysed with FastDigest restriction enzymes in an electrophoretic gel of 1% agarose for the release of the expected insert. Moreover, the purified plasmid was sequenced using universal primers, CMV-F (CGCAAATGGGCGGTAGGCGTG) and WRPE-R (CATAGCGTAAAA GGAGCAACA) (Macrogen, Madrid, Spain) for sequence confirmation.

### Protein production and purification

ExpiFectamine^{™} 293 Transfection Kit (Ref. A14524, ThermoFisher) was used according to the manufacturer's instructions for plasmid transfection and protein production. Briefly, Expi293F^{™} (ThermoFisher) cells at high density (4,5-5,5x106 cells/mL) and with cell viability >95% were diluted to a final density of 3.0×10⁶ cells/mL with pre-warmed Expi293F^{™} Expression Medium (ThermoFisher). Plasmid DNA of sIRs was diluted with Opti-MEM medium (1.0 µg/mL). Then, the ExpiFectamine^{™} reagent was diluted with Opti-MEM medium and was mixed by swirling or inversion. The diluted ExpiFectamine^{™} 293 reagent was added to the plasmid DNA and mixed by swirling or inversion. The ExpiFectamine^{™} and plasmid DNA complexes were incubated at room temperature for 10-20 min. The complexes were slowly transferred to the Expi293F^{™} culture flask gently during addition. Then, the cells were incubated in a 37°C incubator with ≥80% relative humidity and 8% CO₂ on an orbital shaker for 22 hours at 125 rpm. After that, ExpiFectamine^{™} Enhancer#1 and Enhancer#2 were added to the transfection flask and gently swirled during addition. The culture supernatant with the protein of interest was harvested five days post-transfection unless otherwise specified. First, the supernatant was harvested by centrifugation for 5 min at 300 g and then at 1000 g for 10 min. Finally, clarified supernatant was filtered using disposable sterile 0.22 µm filter units. Metal affinity chromatography using HisTrap^{™} Excel columns (Merck) was used to purify the soluble protein following the manufacturer's instructions. The column was equilibrated with at least 5 column volumes (CV) of 500 mM NaCl-PBS (Ref. 10010056, Gibco). The sample was loaded at a flow rate of 5 mL/min. The sample was washed with 20 CV of 500mM NaCl-10mM imidazole-PBS at 5 mL/min. Then, the sample was eluted applying a linear elution gradient (10 to 20 CV) from 10 to 500mM imidazole in 500mM NaCl-PBS at a flow rate of 5 mL/min. Imidazole was removed from the fractions using an Amicon^{®} Ultra-15 Centrifugal Filter Unit (Merck).

### Protein quantification and quality control

Protein concentration was determined by measuring absorbance at 280 nm using a NanoDrop One/One (ThermoFisher Scientific). The purity and integrity of sIR proteins were verified in a 4-20% SDS-PAGE dyed with Imperial Stain (Imperial Protein Stain, Ref. A1435101, Thermo Scientific). Alternatively, the protein specificity was verified via Western Blot using Immun-Blot^{®} Low Fluorescence PVDF/Filter (Ref. 162-0261, Bio-Rad) and 1/1000 purified anti-6-His epitope Tag antibody (clone 6-His, Ref. 906101, Biolegend) with 1/20,000 IRDye^{®} 800CW goat anti-mouse IgG secondary antibody (Ref. 926-32210, Li-Cor) or with 1/10000 HRP-AffiniPure Goat anti-mouse IgG secondary antibody (ref 115-036- 071, Jackson). To perform the SEC-MALS analysis and determine the oligomeric state of different sIR, 100µL of thoroughly purified protein samples at 1mg/mL were loaded on a KW-803 column (Shodex) equilibrated with PBS at 0.9 mL/min flow rate (Shimadzu HPLC system). Detection was performed using a DAWN 8+multi-angle light scattering detector and an Optilab T-rEX differential refractometer (Wyatt Technology) (Protein Technologies Core Facility, CRG). Molar mass was calculated with the Astra 6.1.17 software using a differential index of refraction (dn/dc) value of 0.183 m Ug.

### Protein binding to human and murine CD 155.

To investigate the binding of sIR prototypes to CD155, the inventors employed complementary technologies, including surface plasmon resonance (SPR), functional ELISA and a cell-based assay.

*Protein binding by Surface Plasmon Resonance.* Binding kinetics and affinity for sIR proteins were evaluated by surface plasmon resonance on a BIACORE T100 instrument (GE Healthcare). The assay involved hCD155 and mCD155 Fc chimeras captured on a Series S CM5 chip. Briefly, amine coupling was used to capture a mCD155 Fc chimera (9670-CD, Biotechne) or to create a human IgG surface (anti-human Fc mAb) followed by the capture of hCD155 Fc chimera (9174-CD, Biotechne) according to instructions from the GE Healthcare human IgG capture kit. Each antibody was captured on flow cell 4, leaving flow cell three as a subtractive reference. Capture levels of hCD155 or mCD155 were targeted between 100 and 200 resonance units, after which serial dilutions of sIR proteins were flowed over immobilised hCD155 or mCD155 (30 µL/min for 2 min) and allowed to dissociate for 3 min. The capture surface was regenerated with a 60- s injection of 3M MgCl₂ between each injection. A 5-fold concentration series of each sIR variant ranging from 50 to 0.08 nm was used to analyse binding to hCD155 and mCD155. All sensorgrams were analysed using a 1:1 Langmuir binding model with software supplied by the manufacturer to calculate the kinetics and binding constants. To determine slR2 K_{D} containing a human IgG fragment, direct amine coupling was used to coat the CD155 Fc chimera in the S CM5 chip.

*Protein binding by functional ELISA.* To determine the binding of sIRs to CD155 by ELISA, a 96-well plate was coated overnight with recombinant human and murine CD155/PVR Fc Chimera (9174-CD or 9670-CD; R&D Systems) at a concentration of 2.5 µg/mL in 1x ELISA Coating Buffer (421701, Biolegend). The plate was washed with wash buffer (0.05 PBS/Tween, 5×300µL) and blocked with 300µL of blocking buffer (PBS 3% FBS) for 2 hours. Then, the excess blocking buffer was removed, and the plate was washed three times with 300µL of wash buffer. Subsequently, 100µL of 3-fold serial dilutions of each sIR starting at 200µg/mL were added and incubated at 2-8°C overnight. The following day, the plate was washed, and 100µL of biotinylated 6-His antibody (1:1000, Cat. 906103, Biolegend) were added and incubated at room temperature for 2 hours. Then, the plate was washed, and 100µL of Avidin-HRP conjugate (1:2000, Cat. 405103, Biolegend) were added and incubated at room temperature for 30 min. After another wash, 100µL of chromogenic substrate 1-Step^{™} Ultra TMB-ELISA (Cat. 34028, ThermoFisher) were added, and the plate was incubated at room temperature for 5-10 min. The reaction was stopped by adding 100µL of stop solution (2M H₂SO₄, Sigma Aldrich). The absorbance was read at 450 nm with background subtraction at 540 nm in an Ensight Multimode Plate Reader (Perkin Elmer).

*Protein binding to Bw5147 cell lines.* The T-cell stimulator Bw5147 cell line expressing human CD155 (Bw hCD155) was cultured using previously described methods The cells were harvested with cell viability >95%, and 2×10⁵ live cells were transferred into each well in a 96-well plate with U-bottom. Cells were washed once with PBS after centrifugation at 400g. Then, sIRs were diluted in PBS to generate a 3-fold serial dilution starting at 200µg/mL. After that, cells were pelleted and thoroughly resuspended in 100µL of the sIR serial dilutions. Bw hCD155 cells were incubated at 4°C for 1 hour with the sIRs. After the incubation, the cells were washed once with FACS buffer and incubated in 100µL of PE-anti His antibody solution (5µL per test, Biolegend, Cat. 362603). The staining was incubated at 4°C for 60 min, protected from light and then cells were washed three times with PBS and resuspended in 200µL of PBS. Samples were acquired in a FACSCanto cytometer (BD). The binding of sIRs to CD155 was determined by measuring the MFI of PE in the live cells gated by FSS/SSC. The binding of sIRs to CD155 was assessed by measuring the mean fluorescence intensity (MFI) of PE in the cells gated on FSS/SSC. The IC₅₀ was determined using a sigmoidal, four-parameter logistic (4PL) regression model applied to the normalised log-transformed MFI intensity.

### LCMV chronic infection model and ethics

For the study design, in vivo experiments were performed using C57BL/6J- OlaHsd (C56BL/6, 6-7 weeks of age) maintained under Specific-Pathogen Free (SPF) conditions during the experiment in the bio-contention environment (BSL3) at the Center of Comparative Medicine and Bioimaging (CMCiB-IGTP, Spain). The experimental design of the animal study included 50% sex representation (male, female) to address potential sex-based differences across all the analyses.

The C57BU6 mice were purchased from Envigo Laboratories (Barcelona, Spain). To induce chronic infection in C56BU6 mice, a high dose of focus-forming units (FFU) 1×10⁶ of LCMV_{DOC} was administered by intraperitoneal (IP) injection. The LCMV_{DOC} was grown, stored, and quantified according to previously published methods. The PBS saline injection generated uninfected mice (Mock) as a control study group. All the experimental procedures were approved by the ethical committee for animal experimentation (CEEA-CMCiB, 22-001- MML) and by the local authorities of the Generalitat de Catalunya (Project 11676). The procedure was conducted according to the Guide for the Care and Use of Laboratory Animals provided by the Generalitat de Catalunya, the Principles of Laboratory Animal Care outlined by the National Institute of Health (NIH Bethesda, MA, USA) and the Declaration of Helsinki for animal experimental investigation.

### Study design of LCMV chronic infection

To establish an LCMV model of chronic infection, a group of uninfected (Mock; n=10) and infected mice (LCMV; n=10) were monitored over 28 days after high- dose infection with LCMV_{DOC} strain. The inventors recorded weight measurements over 28 days to evaluate acute and chronic stages of LCMV infection. After euthanasia, on days 14, 21, and 28 (n=4 per group/per time point), necropsies were performed to obtain total blood by cardiac puncture, and the spleen was harvested to evaluate FFU and perform T-cell immunophenotype (Fig. 3A).

### Study design of LCMV chronic infection to test sIRs prototypes efficacy

A proof-of-concept study was carried out using the LCMV mice model of chronic infection to assess the efficacy of the sIR prototypes. Briefly, uninfected (Mock; n=10) and LCMV_{DOC} infected (LCMV; n=80) CL57BU6 mice were monitored for 21 days. On day 21, LCMV_{DOC} mice were randomly allocated to each treatment arm, including LCMV Saline, sIR4, sIR9, αTIGIT, αPD-L1, sIR4+PD-1, sIR9+PD- L1, and αTIGIT+αPD-L1 (n=10 for each group). All IP administrations were conducted using a 29G needle (BD, Spain) and repeated every three days until day 34 post-infection (Fig. 4A). Detailed information regarding the reagents used can be found in Table 1. Blood samples were collected 24 hours before and 24 hours after each IP administration. For this purpose, mice were punctured at the facial vein using a 4-5mm lancet (Novico Medica, Spain) to obtain approximately 20-30 µL of blood, which was then collected in a clotting activator 1.5 mL Microvette^{®} tube (SARSTEDT). Additionally, longitudinal weight measurements were recorded at 2-4 days intervals. Upon euthanasia on days 28 (n=5, for each group) and 34 (n=5, for each group), necropsies were performed, total blood by cardiac puncture was obtained, and spleen and kidneys were harvested for further analysis.

**Table 1. Description of molecules**

| Molecule | Clone | / | IgG | Supplier |
|---|---|---|---|---|
| sIR4 | - | / | Mouse | Genscript |
| | | | IgG2c | |
| | | | ΔPCPP | |
| sIR9 | - | / | Mouse IgG1 | Genscript |
| αTIGIT | 10A7 | / | Mouse | InvivoGen |
| | derived | | IgG2a | |
| αPD-L1 | 10F.9G2 | / | Rat IgG2b | BioXCell |

### Quantification of LCMV by Focus-Forming Assay

The viral titers from the spleens or serum of LCMV-infected mice were assessed using a focus-forming assay (FFA) in MC57 cells. Upon necropsy, half of the spleen was collected in cryotubes and stored at -80°C until required. For serum determination, blood was allowed to be clotted in the Microvette^{®} tube for 1 hour, and serum was recovered by centrifugation at 4,000g for 10 min; samples were subsequently stored at -80°C. To determine the FFU, frozen spleens and serum samples were homogenised, resuspended in DMEM (Invitrogen), and subjected to serial dilutions. Subsequently, 100 µL of each dilution was plated in a 24-well plate (Sigma-Aldrich) containing 2×10⁶ cells/well and incubated for 6 hours at 37°C. Following the formation of a cell monolayer, a 1:1 mixture of 3% Methocel (Sigma-Aldrich) and 2X DMEM 12,5% FBS (Invitrogen) was added to each well and incubated for 48 hours at 37°C. For LCMV antigen staining to determine FFUs, the cells were fixed with 37% formaldehyde (Sigma-Aldrich), washed twice with PBS (Gibco), and incubated for 20min with a 1% TritonX solution (Sigma-Aldrich) for permeabilisation. To minimise nonspecific binding, the cells were incubated for 1 hour at room temperature with PBS containing 10% FBS. Subsequently, the cells were incubated for 1 hour with a rat anti-LCMV mouse antibody (clone VL4, Abyntek) and an additional hour with an anti-rat polyclonal IgG HRP secondary antibody (Jackson ImmunoResearch). The plaques were visualised using the Metal Enhanced DAB Substrate KIT (ThermoScientific). After drying, the spots were counted using an automated ELISpot reader unit (Cellular Technology Limited, Shaker Heights, OH). FFU results from the spleen were normalised by the spleen weight or the serum volume before homogenisation.

### Splenocyte isolation

The protocol for isolating splenocytes from mice spleen involves enzymatic disruption of the extracellular matrix to obtain a single-cell suspension suitable for immunophenotype and *ex vivo* T-cell stimulation. Briefly, half of the spleen was collected in complete RPMI media on ice to maintain cell viability and integrity. The spleens were then aseptically transferred onto a 6-well plate and washed with cold PBS to remove any external debris. Subsequently, 0.5mL of an enzymatic mix containing 20µL/mL DNase (Benzonase, Merck) and 64µL/mL Liberase^{™} TL (Collagenase I/II and Thermolysin, Sigma-Aldrich) was gently injected into the tissue using a pipette tip, ensuring complete perfusion of the spleen with the enzymatic solution. Following this, the organs were carefully disrupted into small pieces and incubated for 20-30 min at 37°C and 5% CO₂ to facilitate tissue digestion and release of cells from the extracellular matrix. After the enzymatic digestion, the disrupted spleen was resuspended in 1mL of RPMI supplemented with 5% FBS and passed through a 70 µm cell strainer to obtain a single-cell suspension of splenocytes. The splenocyte suspension was washed by centrifugation at 400g for 6 min at 4°C. Subsequently, the cell pellet was treated with ACK Lysis buffer (Lonza) for 5 min following the manufacturer's instructions to remove red blood cells. Then, cells were washed by centrifugation at 400g for 6 min at 4°C to discard any remaining tissue debris and resuspended in 10 mL of R10 medium. Cell counting was performed using a NucleoCounter NC-3000 (ChemoMetec), and the cell suspension was diluted to a final concentration of 10⁷ live cells/mL.

### Characterisation of LCMV-specific CD8 T-cells responses in splenocytes

Splenocytes were allowed to rest at 37°C with 5% CO₂ for 1 hour. After resting, cell density and viability were determined, and media was replaced by centrifugation with fresh R10 medium supplemented with 5 µg/mL anti-CD28 (clone 37.51, ThermoFisher) and 20 µL/mL anti-CD107a BV421 (clone 1D4B, Biolegend). Subsequently, cells were cultured in the absence or presence of the immunodominant gp33 CD8 T-cell peptide at 1 µg/mL (GP133-41, Genscript) at a density of 5×10⁶ cells/mL in a 48-well plate. For general TCR stimulation, splenocytes were stimulated with αCD3 (Clone 17A2, FG, ThermoFisher) pre- coated well at 1 µg/mL for TCR stimulation. Then, cells were incubated at 37ºC with 5% CO₂ overnight. The next day, protein transport inhibitors, Brefeldin A (BD Biosciences) and Monensin (BD Biosciences) solutions, were added to the cell culture and incubated for 3 hours at 37ºC with 5% CO₂ before harvest for immunophenotype by flow cytometry.

### Immunophenotype by flow cytometry

For the immunophenotype of lymphoid and myeloid cells, 2×10⁶ splenocytes were rested in R10 for 1 hour at 37ºC and 5% CO2. Then, splenocytes were harvested after overnight incubation in the absence or presence of gp33 peptide before staining. Briefly, splenocytes were labelled with a Live/Dead probe (LIVE/DEAD^{™} Near-IR Dead Cell Stain, Invitrogen) and washed twice with Flow Buffer (PBS, 5% FBS). Following the manufacturer's indications, the Fc-receptors were blocked with CD16/CD32 (Mouse BD Fc Block^{™}). Splenocytes were then stained for T-cell immunophenotype with the surface markers CD3 (BV570 clone 17A2, Biolegend), CD4 (A647 clone GK1.5, Biolegend), CD8b (A700 clone 53-6.7, Biolegend), Nk1.1 (BV605 clone PK136, Biolegend), CD19 (PE/Dazzle clone 6D5, Biolegend), CD27 (BV785 clone LG.3A10, Biolegend), CD11b (BV510 clone M1/70, Biolegend), PD-1 (PE clone 29F.1A12, Biolegend) and TIGIT (PECy7 clone 1G9, Biolegend). For myeloid immunophenotyping, B220 (APC-Fire750 clone RA3-6B2, Biolegend), Ly6G (APC-Fire750 clone 1A8, Biolegend), SiglecF (APC Cy7 clone S17007L, Biolegend), CD3 (APC-Fire750 clone KT3.1.1, Biolegend), NK1.1 156516 (APC-Fire750 clone S17016D, Biolegend), MHCII (BV421 clone M5/114.15.2, Biolegend), CD11b (BV510 clone M1/70, Biolegend), CD11c (A700 clone N418, Biolegend), CD86 (A647 clone GL-1, Biolegend), CD40 (PE Dazzle 594 clone 3/23, Biolegend), hTIGIT (PE-Cy7, clone MBSA43 ThermoFisher), Ly6c (BV570 clone HK1.4, Biolegend), CD155 (BV605 clone TX56, Biolegend), PD-L1 (BV786 clone 10F.9G2, Biolegend) were employed. Subsequently, cells were washed, fixed and permeabilised with a Fix&Perm kit (ThermoFisher) for intracellular cytokine staining with IFNy (BV711 clone XMG1.2, Biolegend), TNF (BB700 clone MP6-XT22, BD), IL6 (FITC clone MP5-20F3, ThermoFisher), IL10 (BV650 clone JES5-16E3, BD) antibodies. Then, samples were fixed in 1% formaldehyde and acquired on BD LSRFortessa ^{™} (Beckmann Coulter) using the FACS DiVa software (BD, Biosciences). Data were analysed by FlowJo v10.6 (Tree Star Inc). To determine the frequency of the different populations and functional markers, the inventors employed Fluorescence Minus One (FMO) control. The frequency of αCD3 and gp33-specific T-cell responses was determined by subtracting the percentage of cytokine production from the unstimulated control to the αCD3 or gp33 stimulation. The inventors considered a positive gp33-specific T-cell response after background subtraction used as a cut- off value and a minimum of 400 events in the gate of cytokines.

### Multiplex cytokine assay

The LXSAMSM-17 Mouse Luminex^{®} Discovery Bead panel (Bio-Techne) was used to quantify the following analytes: BAFF, IFNγ, TNF, IL-2, IL-4, IL-5, IL-6, IL- 10, IL-13, IL-27, IL-33, TNFRI, MIP-1a, CCL2, CCL4, Granzyme B and RANTES, in serum samples collected before administration of immune intervention at day 21 post-infection and after 3 (day 28) and 5 (day 34) doses, following the manufacturer's instructions. Plates were then read by Luminex 200, Austin Luminex, USA. Data were analysed using MILLIPLEX Analyst 5.1 software (Merck Millipore Darmstadt, Germany). Values of analytes after 3 and 5 doses were transformed in Fold Change (FCh) using day 21 as a baseline.

### Kidney pathology

Once mice were euthanized, at the two end-points (day 28 and day 34), kidney were harvested, embedded in optimal cutting temperature compound (in Tissue-Tek^{®} O.C.T.^{™}) and stored at -80°C until fixation.

Then, kidneys were unfrozen, washed with PBS, fixed using neutral buffered formalin, included in paraffin and cut in 3 µm slices using a Leica 2255 microtome. Slices were stained with hematoxylin-eosin, observed with an Olympus BX45 microscope, and inflammation scores, ranking from 1 (not damage) to 4 (most inflammation), were automatically determined by the PathData System V6.2e1 software. Both kidneys from 3 animals per group were analyzed and the higher score for each mice was used for comparison (Fig. 19).

### Statistics

One-way ANOVA analyses were performed with Fisher's LSD test employed for multiple comparisons or among study groups. Two-way ANOVA comparison was performed with Fisher's LSD test for multiple comparisons among study groups at different time points. The analysis was conducted using GraphPad Prism (v10) software. The p-values (p) below 0.05 were considered significant and were indicated by asterisks as follows: *p ≤ 0.05; **p ≤ 0.01; ***p ≤ 0.001; ****p ≤ 0.0001. Non-significant differences were indicated as "ns".

### Results

### Example 1 - In silico design and modelling of sIR prototypes

To design sIR recombinant proteins targeting CD155, the inventors identified the relevant domains of the human TIGIT inhibitory receptor using the information recapitulated in the Universal Protein Resource (UniProt) database (TIGIT - Q495A1). In the UniProt server, the more basic classification of the TIGIT membrane protein is described with the extracellular, transmembrane and intracellular regions (Fig. 1A).

To generate soluble inhibitory receptors (sIRs) capable of interacting with CD155, the inventors preserved the extracellular domains of the TIGIT inhibitory receptor, which include the native signal peptide (SP Nat), the Ig-like V-type domain and the 17 amino acids tail located prior to the transmembrane domain. In the Ig-like V-type domain of TIGIT, the inventors can identify two critical functional sequences: 1) the homodimerization domain, which facilitates the interaction of two TIGIT subunits in cis and 2) the three aminoacidic sequences (VTQ, AX6G, and TYP) that allow the heterotetrameric interaction with CD155 in trans (Fig. 1A). Next, to obtain the first soluble prototype, slR1 Nat, the sequences of a BamHl spacer (GS), a His- tag sequence (HHHHHH) (SEQ ID NO: 44) and the stop codon (STOP) were added to the construct (Fig. 1B). In addition to the slR1 Nat prototype, the inventors designed two additional prototypes to enhance soluble protein production. These prototypes incorporated different signal peptides (SP) to improve protein secretion. Specifically, the inventors utilised the signal peptide from the T cell surface glycoprotein CD5 (SP CD5) and the signal peptide from the azurocidin preprotein (SP Azu), resulting in slR1 CD5 and slR1 Azu, respectively. In both slR1 CD5 and slR1 Azu, the inventors shortened the 17-amino acid tail by removing the hydrophobic residues that could potentially hinder secretion and promote membrane retention (Fig. 1C).

Then, the different monomeric sIR prototypes were modelled using the iterative I-TASSER tool to study the folding and confirm the preservation of human TIGIT secondary and tertiary structures (Fig. 1D). The inventors confirmed that the homodimerization domains of sIR proteins were conformationally accessible to allow homodimerization and that the VTQ, AX6G, and TYP sequences were exposed to allow interaction with CD155. In addition, the heterotetrameric blockade between two slR1 molecules and two surface molecules of CD155 was modelled to suggest the mode of action of CD155 blockade by the slR1 homodimer. To identify the most efficient monomeric slR1 prototype for transient protein production using the Expi293F system, the inventors transfected plasmids encoding slR1 Nat, slR1 CD5, and slR1 Azu. Seven days after transfection, the inventors observed that slR1 Nat did not result in the production or secretion of soluble protein into the cell culture supernatant. However, the inventors obtained high protein yields of slR1 CD5 and slR1 Azu after transfection (Fig. 1D). These results suggest a suboptimal soluble production using the native signal peptide in the Expi293F system or a significant impairment in the secretion of slR1 prototypes due to the 17-amino acid tail. Moreover, a time course analysis revealed that slR1 Azu transfection produced more protein at 4-5 days post-transfection than slR1 CD5 (Fig. 1E). Based on these results, the inventors decided to maintain the design of slR1 containing the SP Azu to produce the monomeric slR1 prototype and as a scaffold for the subsequent sIR prototypes (Table 2).

**Table 2. Summary sIR prototypes design**

| Human | Ig-like V-type domain | | |
|---|---|---|---|
| sIR1 | human | -- | -- |
| sIR2 | human | hIgG1 | Effector |
| sIR10 | human | hIgG4 S228P | No Effector |
| Hybrid | | | |
| sIR3 | human | mIgG2c | Effector |
| sIR4 | human | mIgG2c ΔPCPP | Effector |
| sIR9 | human | mIgG1 | No Effector |
| Murine | | | |
| sIR5 | murine | -- | -- |
| sIR6 | murine | mIgG2c | Effector |
| sIR7 | murine | mIgG2c ΔPCPP | Effector |
| sIR8 | murine | mIgG1 | No Effector |

Next, the inventors aimed to increase slR1 binding affinity for the CD155 ligand by developing stable sIR dimers that facilitate the homodimerization and heterotetrameric interaction between the sIR prototype and the CD155 ligand. To accomplish this, the inventors introduced Fc-immunoglobulin regions at the C-terminal of the slR1 prototype. Specifically, the inventors used sequences from the effector human IgG1 Fc region to generate slR2 and the non-effector human IgG4 Fc region with the S228P mutation to generate slR10 (Fig. 2A). Additionally, the inventors developed three equivalent sIR constructs to target the mouse CD155 receptor by using the murine TIGIT Ig-like V-type domain, resulting in the monomeric murine sIR5, dimeric slR6 (murine effector IgG2c), and dimeric sIR8 (murine non-effector lgG1) prototypes (Fig. 2B). However, the in-silico comparison of slR2 and slR6 revealed a potential impairment in protein folding of the murine Ig-like V-type domain due to the presence of a PCPP sequence in the murine IgG2c hinge region. To compensate for the potential incorrect folding, the inventors designed the slR7 prototype with a deletion of the PCPP amino acids (ΔPCPP). Moreover, based on studies that support the binding of the human Ig-like V-type TIGIT domain to both human and murine CD155, the inventors designed hybrid sIR prototypes incorporating the human Ig-like V-type domain and murine Fc IgG regions, resulting in sIR3, slR4 and slR9 (Fig. 2D). Finally, the inventors designed a human Fc-Control (Fc-Ctrl) protein, deleting the Ig-like V-type domain as a control molecule (Fig. 2E).

### Example 2 - Production, purification and quality control of sIR proteins

Next, the inventors transfected the encoding plasmids into the Expi293F cell culture system and obtained recombinant soluble proteins. Following supernatant harvest and protein purification by metal affinity chromatography, the inventors assessed the purity and molecular weight (MW) of all the sIR prototypes using SDS-PAGE and Western blot (WB) analysis. The inventors confirmed purity <95% and expected theoretical MW by SDS-PAGE and confirmed the presence of the His-tag in the C-terminal region by WB for all prototypes (Fig. 5 A-B).

In addition, the inventors employed SEC-MALS to evaluate the protein oligomerization state of sIR prototypes. Despite the potential of slR1 and slR5 to form homodimers in solution, due to the presence of the homodimerization domains, the inventors only detect monomeric forms by SEC-MALS. Moreover, SEC-MALS confirmed the stable dimeric state of the dimeric sIR prototypes and suggested potential glycosylation during protein production due to the difference between theoretical MW and SEC- MALS MW determination (Table 3).

**Table 3. Theoretical and empirical molecular weight of sIRs**

| | Prototype | Theoretical Monomer | Theoretical Dimer | SEC-MALS | Structure |
|---|---|---|---|---|---|
| | | MW (KDa) | MW (KDa) | MW (KDa) | |
| Human | sIR1 | 12.8 | -- | 17.9 | Monomer |
| | sIR2 | 40.4 | 80.8 | 94.7 | Dimer |
| | sIR10 | 40.6 | 81.2 | 95.2 | Dimer |
| Hybrid | sIR3 | 40.6 | 81.2 | 94.5 | Dimer |
| | sIR4 | 40.2 | 80.5 | 91.4 | Dimer |
| | sIR9 | 40.5 | 81.0 | 107.8 | Dimer |
| Murine | sIR5 | 12.7 | -- | 17.6 | Monomer |
| | sIR6 | 40.5 | 81.0 | 92.7 | Dimer |
| | sIR7 | 40.0 | 80.0 | 91.3 | Dimer |
| | sIR8 | 39.1 | 78.2 | 97.2 | Dimer |

### Example 3 - Dimeric sIRs bind with high affinity to hCD155 and mCD155 ligands

After confirming the purity and conformational state of the sIRs, the inventors assessed binding and affinity for the CD155 ligand, including both human and murine CD155 variants using BIACORE Surface Plasmon Resonance (SPR) analysis, functional ELISA and a cell-based assay.

First, the inventors observed a low affinity of slR1 and slR5 for mCD155 by BIACORE assay. The low affinity of the monomeric sIRs was attributed to the rapid association and dissociation rate from mCD155, which hindered the experimental determination of the K_{D} affinity value (Fig. 6A-C and Table 4). However, the stable dimeric prototypes exhibited significantly slower dissociation rates from mCD155, resulting in a K_{D} value in the nM range (Fig. 6A-C and Table 4). When comparing the K_{D} of dimeric prototypes, the inventors observed similar K_{D} ranges for mCD155, with median K_{D} values of 45.4 nM, 46.5 nM, and 76.1 nM for the human, hybrid, and murine prototypes, respectively, indicating a good cross- species recognition between the human and murine Ig-like V-type domains for murine CD155 (Table 4).

**Table 4. Affinity constant for siRs to hCD155 and mCD155**

| | mCD155 K_{D}(nM) | hCD155 K_{D}(nM) |
|---|---|---|
| Human | | |
| sIR1 | UND | UND |
| sIR2 | 42.2 | 8.64* |
| sIR10 | 48.5 | -- |
| Hybrid | | |
| sIR3 | 33.9 | 1,65 |
| sIR4 | 50.2 | 0,63 |
| sIR9 | 55.4 | 2,48 |
| Murine | | |
| sIR5 | UND | UND |
| sIR6 | 77.1 | UND |
| sIR7 | 55.8 | UND |
| sIR8 | 95.3 | UND |

| | | |
|---|---|---|
| K_{D}: Affinity constant; UND: Undetermined; * Determined by direct amine coupling of hCD155 | | |

On the other hand, the BIACORE assay conducted to determine the affinity of sIR for hCD155 indicated a similar rapid dissociation rate of the monomeric slR1 and slR5 from hCD155. For dimeric sIRs, the inventors detected a high binding affinity of human and hybrid sIRs for hCD155, with a K_{D} of 8.64 nM for slR2 and a median K_{D} of 1.7 nM for the hybrid sIRs (Table 4). However, the inventors observed weak affinity of dimeric murine sIR6, sIR7, and slR8 prototypes for hCD155, consistent with previous studies, indicating scarce recognition of hCD155 by the murine Ig-like V-type domain. Moreover, a comparison of BIACORE results for mCD155 and hCD155 ligands revealed consistently higher K_{D} values in human and hybrid sIRs for mCD155 (ranging from 55.4nM to 42.2nM) compared to hCD155 (ranging from 8.64nM to 0.63nM). Furthermore, when investigating the impact of the ΔPCPP mutation in the murine IgG2c hinge region, the inventors obtained inconclusive results. In the BIACORE assay with mCD155, the deletion increased the K_{D} in the hybrid slR3 from 33.9 to 50.2 nM in the modified sIR4, while it decreased the K_{D} in the murine slR6 from 77.1 to 55.8 nM in the modified sIR7. However, in the BIACORE assay with hCD155, the ΔPCPP deletion decreased the K_{D} in the hybrid slR3 from 1.65 to 0.63 nM in the modified slR4 (Table 4). These results indicate a limited impact of the isotype Fc fraction employed on the K_{D} values.

Additionally, the inventors investigated the binding affinity of the sIR prototypes to human and murine CD155 by functional ELISA. In the functional ELISA for hCD155 across prototypes, the inventors did not obtain a binding curve for monomeric slR1 due to the low signal within the same concentration range (Fig. 7A). In the case of dimeric human and hybrid sIRs, the inventors observed similar binding curves for hCD155 with a median IC₅₀ of 24.8 ng/mL (Table 5). Similar findings were obtained in the functional ELISA for mCD155 across prototypes with dimeric prototypes binding with higher affinity than slR1 (Fig. 7B). Overall, the IC₅₀ values obtained from functional ELISA were higher for mCD155 (median 220 ng/mL) compared to hCD155 (median 24.8 ng/mL) (Table 5). These findings are consistent with the results obtained from the SPR study, which demonstrated higher K_{D} values for the human and hybrid sIRs when binding to mCD155 compared to hCD155. These higher K_{D} and IC₅₀ values indicate a weaker binding affinity of the human and hybrid sIRs for mCD155.

**Table 5. IC₅₀ for sIRs binding to mCD155 and hCD155 determined by functional ELISA.**

| Human | mCD155 IC50 (ng/mL) | hCD155 IC50 (ng/mL) |
|---|---|---|
| sIR1 | UND | UND |
| sIR2 | 204.5 | 33.65 |
| sIR10 | 66.13 | 11.96 |
| Hybrid | | |
| sIR4 | 235.5 | 16.04 |
| sIR9 | 304.1 | 57.25 |

| | | |
|---|---|---|
| UND; undetermined | | |

Finally, the inventors assessed the binding of human sIR to hCD155 using a cell-based assay in Bw5147 cell lines (Bw hCD155 and Bw Ctrol). As shown in Fig. 7C, the inventors confirmed the specific binding of slR2 and sIR10 to Bw hCD155 compared to Bw Ctrol cells. In addition, the IC₅₀ values obtained in the cell-based assay for slR2 and slR10 (19.15ng/mL and 26.87 ng/mL, respectively) were consistent with the values obtained in the functional ELISA further confirming the binding of the human dimeric sIRs to hCD155 within the range of 10-30 ng/mL.

Taking the results from this section, the inventors confirmed: 1) the limited binding of monomeric slR1 and slR5 prototypes to CD155 ligands; 2) the suitable cross- species recognition with similar K_{D} values of human, hybrid and murine sIRs to mCD155, but limited recognition of murine sIRs to hCD155; 3) the consistent higher affinity of human and hybrid sIR prototypes for hCD155 in comparison to mCD155, and 4) the limited impact of the different Fc regions included in the design of dimeric sIR prototypes in the K_{D} or IC₅₀ values.

### Example 4 - LCMV infection at high doses induces chronic infection and viral persistence.

First, the inventors conducted an *in vivo* study in the LCMV mice model to evaluate the expression of IRs (TIGIT and PD-1) and functional changes in T-cell immune responses during the chronic infection stage. Briefly, C57BL/6 mice received a high dose (10⁶ FFU) of LCMV_{DOC} strain to induce chronic infection (LCMV, n=12) and compared with uninfected control mice (Mock, n=12). The inventors performed necropsies (n=4, per group) on days 14, 21, and 28 to collect total blood and harvest spleen for FFU assay and immunophenotyping of CD8+ and CD4⁺ T cells and NK cells (Fig. 3A). During the follow-up, the inventors assessed weight variation to indirectly characterise acute and chronic phases of LCMV infection.

The inventors observed a significant weight loss in the LCMV-infected mice 7 days post-infection, which gradually recovered from day 9 onwards (Fig. 3B). Cross-sectional analysis showed significant weight loss differences by day 8 between Mock and LCMV-infected mice (Fig. 3C). After the acute infection phase, the inventors observed a progressive weight increase from 8 to 15 days. Despite this progressive weight increase, significant differences in weight between chronically LCMV-infected mice and Mock mice persisted until day 28 (Fig. 3C), indicating chronically LCMV-infected mice cannot fully recover weight within three weeks from the onset of infection. Furthermore, the inventors evaluated weight variation stratified by sex with similar findings (Fig. 3D). Females tend to gain less weight than males in Mock and LCMV-infected groups. Interestingly, on day 28, no differences were found in female Mock and LCMV-infected groups. The reduced number of mice after stratification by sex can be a limitation in assessing weight variation over the study period.

Next, the inventors analysed FFUs in the spleen and serum samples collected after necropsies (Fig. 8). Our findings revealed viral persistence in both spleen and serum at all three evaluated endpoints. However, the FFU/g values in the spleen were higher compared to the FFU/mL in serum samples, as expected (Fig. 8A). The viral persistence in the spleen ranged from 10⁴ to 10⁵ FFU/g on day 14 and 21 post-infection and decreased to 10³-10⁴ FFU/g on day 28 post-infection. On the other hand, the viral load in serum started at 10⁴ FFU/mL on day 14 and decreased to 10² - 10³ FFU/mL on day 28 post-infection. Due to the assay detection limits, the inventors could not quantify FFUs in two spleen and three serum samples. The inventors did not find differences by sex regarding FFU levels, and undetectable samples were distributed across male and female mice (Fig. 8B). These results suggest that sex is not a variable relevant to viral persistence levels in this model.

These findings indicate that LCMV infection persists for at least 28 days after the acute infection phase, as evidenced by detecting LCMV FFU in both serum and spleen. The significant decrease in weight observed at 7 days post-infection indicates the severity of the acute phase of infection. Remarkably, the weight in LCMV-infected mice was gradually recovered over the 28 days assessed, although it did not reach the levels of uninfected mice. Notably, the partial weight recovery after the acute phase, accompanied by LCMV persistence in serum and spleen, indicated a chronic infection phase established from day 21-28.

### Example 5 - LCMV chronic infection induces irreversible PD-1 and TIGIT expression in T-cells and loss of T-cell functionality

Then, to comprehensively evaluate the cellular immune responses during chronic LCMV infection, the inventors monitored changes in CD4+, CD8+ T-cells and NK cells in splenocytes over time. The inventors performed immunophenotype in splenocytes 18 hours after spleen processing and stimulation. In unstimulated conditions, the inventors observed changes regarding cell dynamics with a significant decrease in CD8⁺ T cells and an increase in CD4⁺ T cells comparing infected and uninfected mice (Fig. 9 A-B). Intriguingly, this alteration persisted for at least 28 days post-infection. In contrast, no significant changes were observed in the NK cell population frequencies on days 21 and 28, maintaining a frequency of around 1% in splenocytes.

Next, to assess CD8 T-cell exhaustion during chronic LCMV infection, the inventors examined the expression of PD-1 and TIGIT in T-cells after stimulation under two conditions: 1) αCD3 (TCR stimuli) and 2) gp33 peptide (LCMV antigen) and compared to unstimulated Mock and LCMV-infected splenocytes. Our results indicate that LCMV infection upregulated the expression of PD-1 in CD8⁺ T-cells compared to Mock, with the highest expression on day 14 and significantly decreasing on 21 and 28 days post-infection (Fig. 10A). Notably, PD-1 upregulation in unstimulated CD8⁺ T-cells from infected mice persisted at a median of 26% compared to Mock at 28 days (Fig. 10A). Similar results were found in αCD3 stimulation. Remarkably, roughly 91% of CD8⁺ T-cells in LCMV- infected mice expressed PD-1 during αCD3 stimulation on day 14, decreasing to 65% on days 21 and 28, corroborating the irreversible induction and maintenance of PD-1 expression in CD8⁺ T-cells during chronic infection (Fig. 10A).

On the other hand, LCMV infection induced TIGIT expression in CD8⁺ T-cells, with a peak expression of 9.5% TIGIT+CD8⁺ T-cells on day 14 and an irreversible increase for up to 28 days compared to uninfected Mock mice (Fig. 10B). Upon αCD3 stimulation, TIGIT+CD8⁺ T-cells were significantly increased by up to 40% on days 21 and 28 in LCMV-infected mice (Fig. 10B). Interestingly, TIGIT+CD8⁺ T-cells were barely detectable upon stimulation in Mock mice (Fig. 10B).

Notably, most TIGIT+CD8⁺ T-cells co-expressed PD-1 in unstimulated and stimulated experimental conditions. Moreover, when stimulated with gp33 peptides, there was an increased frequency of double-positive PD-1+TIGIT+ CD8⁺ T-cells compared to unstimulated conditions (median 6.2% and 2.4%, respectively), indicating that the expression of TIGIT is motivated by antigenic stimulation in this context (Fig. 10C). Remarkably, the absence of double-positive PD-1+TIGIT+ CD8⁺ T-cells in Mock mice, independent of any stimulation, indicates the relevant role of PD-1 and TIGIT co-expression in LCMV chronic viral infection.

Then, the inventors evaluated changes in the expression of PD-1 and TIGIT in CD4⁺ T-cells. LCMV infection significantly increased PD-1 expression in unstimulated CD4⁺ T-cells compared to Mock at 14, 21, and 28 days. Interestingly, in the absence of stimulation, the levels of PD-1 expression were higher in CD4⁺ T-cells than in CD8⁺ T-cells (Fig. 10A-11A). Moreover, during LCMV infection, the inventors noted a decrease in PD-1+CD4⁺ T-cells between days 14 and 21, followed by an increase in PD-1+CD4⁺ T-cells between 21 and 28 (Fig. 11A). Similar results were obtained during αCD3 stimulation of CD4⁺ T-cells from LCMV-infected mice compared with Mock mice, with a high frequency of PD-1+CD4⁺ T-cells remaining elevated at 86% (Fig. 11A). On the other hand, the expression of TIGIT was induced by chronic LCMV infection in unstimulated CD4⁺ T-cells, as compared to Mock mice (Fig. 11B). Similar to CD8⁺ T-cells, TIGIT expression remained elevated in unstimulated CD4⁺ T-cells even 28 days post- infection (Fig. 11B). Notably, upon αCD3 stimulation, the frequency of TIGIT+CD4⁺ T-cells increased up to 27% on days 21 and 28, whereas in Mock mice, it remained at 10% (Fig. 11B). Similarly, to CD8⁺ T-cells, the vast majority of TIGIT+CD4⁺ T-cells co-expressed PD-1. However, contrary to CD8⁺ T-cells, the stimulation with gp33 peptides did not alter the frequency of double-positive PD-1+TIGIT+ CD4⁺ T-cells compared to unstimulated conditions (Fig. 11C). Interestingly, after αCD3 stimulation, PD-1+TIGIT+ CD4⁺ T-cells were also present in uninfected mice, indicating that TIGIT expression may be involved in other biological aspects unrelated to LCMV chronic infection in CD4⁺ T-cells.

In summary, CD4⁺ T-cells exhibited elevated PD-1 and TIGIT co-expression frequency in unstimulated and αCD3 conditions compared to CD8⁺ T-cells. Conversely, the co-expression of TIGIT and PD-1 was specifically displayed in CD8⁺ T-cells upon αCD3 and gp33 stimulation. These results indicate an irreversible expression of PD-1 and TIGIT in T-cells motivated by chronic LCMV infection with certain differences in the expression profiles between CD8⁺ and CD4⁺ T-cells.

Then, the inventors evaluated CD8⁺ T cell function in the presence of αCD3 and gp33 based on the CD107a, IFNy and TNF markers. Remarkably, after αCD3 stimulation, the inventors observed a decline in the production of IFNy and TNF in CD8⁺ T- cells from day 14. In addition, the inventors noticed a decrease in the degranulation marker in CD8⁺ T-cells between days 14 and 21, followed by a recovery between days 21 and 28 (Fig. 12A).

Then, the inventors evaluated the LCMV-specific CD8+ T-cell responses directed to the immunodominant gp33 peptide; the inventors observed a decrease in the expression of the CD107a degranulation marker and the production of TNF from day 14 to 21 in gp33-specific CD8+ T cells, which persist until day 28. The decrease in CD107a degranulation marker and TNF production indicates a potential impairment in the cytotoxic activity of gp33-specific CD8+ T cell responses in chronic infection. However, no changes in the production of IFNy were detected (Fig. 12B). These findings align with previous reports indicating early exhaustion of IFNy+ gp33- specific CD8⁺ T-cell responses compared to TNF and CD107a. As previously reported, the gp33-specific IFNγ+CD8⁺ T-cells peak at 7-8 days after LCMV_{DOC} infection, accounting for up to 10% of total CD8+ T-cells. However, IFNy+ gp33- specific CD8⁺ T-cell responses decrease to 3% from day 10 due to T-cell exhaustion in this animal model. Thus, the observed low levels of IFNy+ gp33-specific CD8⁺ T-cell responses from 14 (~4%) represent cells already impaired due to early T-cell exhaustion.

Overall, these results provide further evidence of T-cell exhaustion in the LCMV mice model, as shown by the irreversible coexpression of PD-1 and TIGIT, viral persistence in tissue, and decreased T-cell function. These findings indicate that the model of chronic LCMV infection can recapitulate the immune features of chronic infection and immune exhaustion, being well-suited for evaluating the effectiveness of sIRs prototypes targeting CD155. Additionally, there is potential to explore the combined use of sIR prototypes with αPD-L1, which has been previously shown to recover exhausted T-cell responses in this model, supporting the combinatorial targeting of CD155 and PD-L1 for immunotherapeutics to restore immunity in chronic viral infections.

### Example 6 - slR4 reduces αPD-L1 immune-related toxicity and decreases viral persistence in combinatorial treatment

After characterising the LCMV chronic infection model, the inventors conducted a proof-of- concept safety and efficacy study to evaluate the immunotherapeutic effect of sIR prototypes in blocking the TIGIT/CD155 axis alone or in combination with αPD- L1. The study design involved infected mice receiving a high dose of LCMV_{DOC} strain to induce chronic LCMV infection (LCMV, n=80) and uninfected control mice (Mock, n=10), described in the previous section. On day 21, LCMV-infected mice were separated into the following study arms: single treatment (slR4, sIR9, αTIGIT and αPD-L1) or combined treatments (sIR4+αPD-L1, sIR9+αPD-L1, αTIGIT+αPD-L1) every three days for two weeks (n=10 for each group). The Mock and LMCV control groups received saline administration at the same time points (n=10 for each group). The study design is represented in Fig. 4A. Briefly, the inventors monitored weight and collected blood samples during treatment administration. Moreover, the inventors evaluated viral persistence and characterised immune responses in the spleen after necropsies on mice receiving 3 (day 28) or 5 doses (day 34), respectively.

During the 34-day follow-up, the inventors monitored weight variation to assess safety and toxicity by determining the effect of the immune intervention on weight loss. The inventors observed that LCMV-infected mice experienced significant weight loss 9 days post-infection compared to Mock but gradually recovered from day 10 onwards, consistent with our previous data (Fig. 4B and 3B). Moreover, the inventors conducted a cross-sectional analysis on days 27 and 33 in mice after 3 and 5 treatment doses, respectively. On day 27, the inventors observed a significant weight loss in LCMV-infected mice compared to Mock. No differences were observed between LCMV-infected mice and those treated with sIR4, sIR9, αTIGIT, sIR4+αPD-L1 and αTIGIT+αPD- L1 at day 27. However, the inventors noticed a significant weight loss in animal groups that received single αPD-L1 treatment or combinatorial sIR9+αPD-L1 compared to LMCV-infected mice at day 27 (Fig. 4C). On day 33, single αPD-L1 and all combinations of αPD-L1, except sIR4+αPD-L1, demonstrated a significant weight loss compared with LCMV-infected mice (Fig. 4C). These results suggest that the inclusion of αPD-L1, either as a standalone or in combinatorial therapy, may lead to significant weight loss as a potential measure of immune-related toxicity. Nonetheless, using slR4 in combination with αPD-L1 improved the tolerability of αPD-L1 treatment.

Next, the inventors conducted a viral persistence study to determine FFUs in the spleen collected after necropsies (Fig. 4D). Our findings indicate a significant decrease in FFU in the spleen for sIR4+αPD-L1 on day 28 compared to slR4 or αTIGIT single treatments (Fig. 4E). However, the inventors did not observe significant differences in FFU between any other arm. Notable, FFUs were below the limit of detection (LOD range, 328-1336 FFUs/g) in 3 spleens from αTIGIT treated mice. On day 34, the inventors only found a trend for sIR4+αPD-L1 and a significant reduction of FFUs for sIR9+αPD-L1 treatment compared to the LCMV-infected group Fig. 4E. Remarkably, these results indicate a consistent reduction of LCVM persistence in the spleen for sIR4+αPD-L1 treatment, indicating a potential better immune control of chronic LCMV infection with improved tolerability of this combination.

### Example 7 - αPD-L1 treatment induces immune activation of DCs in single and combinatorial treatment with αTIGIT and sIR4.

In addition, the inventors performed immunophenotype of myeloid and lymphoid compartments in splenocytes obtained after enzymatic tissue processing on days 28 and 34, shown in Fig. 4A. This allowed us to characterise the expression of TIGIT, PD-1 and their ligands, CD155 and PD-L1 and determine the levels of immune activation in the myeloid compartment based on CD40 and CD86.

In the myeloid compartment, the inventors did not observe any changes in the frequency of dendritic cells (DCs) among uninfected mice, LCMV-infected mice, or LCMV-infected and treated mice on day 28. However, the inventors observed a reduction in the frequency of DCs on day 34 between Mock and LCMV-infected mice, which was recovered by αTIGIT treatment (Fig. 13A).

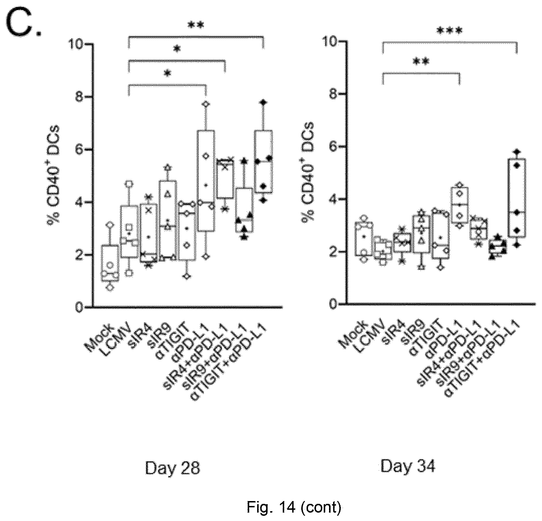

Additionally, the inventors observed a significant decrease in the frequency of PD-L1+ DCs in single or combinatorial treatments containing αPD-L1 compared with LCMV-infected mice on days 28 and 34 (Fig. 14A). Nonetheless, the reduction in PD- L1+ expression in DCs is consistent with antibody blockade and competition, as the inventors used the same clone (10F.9G2) for blocking and PD-L1 staining. On the other hand, the inventors did not observe a reduction in CD155+ expression in DCs across groups containing sIRs. Nevertheless, the inventors found an increase in CD155+ DCs following the administration of αPD-L1 on day 28 and in αPD-L1 and αTIGIT+αPD-L1 on days 28 and 34 (Fig. 14B). Additionally, the inventors did not observe any hTIGIT+ DCs. Finally, when the inventors analysed DC activation using CD40 and CD86 markers, the inventors observed an increase in the frequency of CD40+ DCs in αPD-L1 and αTIGIT+αPD-L1 on days 28 and 34, as well as in sIR4+αPD-L1 on day 28 (Fig. 14C) in the absence of any impact in the frequency of CD86 or CD40/CD86 coexpression.

These findings suggest that PD-L1 blockade was effectively accomplished in DCs. Additionally, the upregulation of CD155 expression in DCs following αPD-L1 and αTIGIT+αPD-L1 treatment supports the rationale for targeting the PD-L1 and TIGIT/CD155 axis in combination. However, the increase of DC activation due to combinatorial immune interventions seems to decrease over time.

### Example 5 - Increased frequency of CD4+ T-cells and reduction of NK cells in chronic LCMV are not reversed by targeting the PD-L1 and TIGIT/CD155 axis.

Then, the inventors analysed the lymphocytic compartment, including CD4+ and CD8+ T- cells and NK cells. Consistent with our prior study, the inventors observed an increase in the frequency of CD4⁺ T-cells and a decrease in CD8⁺ T-cells in LCMV-infected mice compared to uninfected mice on days 28 and 34 (Fig. 15A). Remarkably, no immunotherapeutic interventions tested could reverse these alterations. Furthermore, the inventors observed a general decline in the frequency of NK cells with LCMV infection on days 28 and 34. This reduction in NK cells was increased in the presence of αPD-L1 treatment by day 34 (Fig. 15A-B).

### Example 9 - Increased levels of PD-1 and TIGIT expression in CD8⁺ and CD4⁺ 7 cells in chronic LCMV are not reversed by targeting the PD-L1 and TIGIT/CD155 axis.

Then, the inventors characterised the expression of PD1 and TIGIT in CD8⁺ and CD4⁺ T-cells across study arms. Our findings indicate that chronic LCMV infection significantly increased PD-1 and TIGIT expression in CD8⁺ T-cells on days 28 and 34. In addition, the frequency of TIGIT in CD8⁺ T-cells was significantly reduced in mice treated with αTIGIT monoclonal antibodies, consistently with receptor blockade (Fig. 16A). Interestingly, treatments containing αPD-L1 tended to increase the frequency of PD-1 expression in CD8⁺ T-cells, with statistical significance on day 28 for sIR9+αPD-L1 and αTIGIT+αPD-L1 and on day 34 for αPD-L1 and αTIGIT+αPD-L1 (Fig. 16A). Additionally, αPD-L1 treatment significantly increased TIGIT expression in CD8⁺ T-cells on day 34 compared to LCMV-infected mice (Fig. 16A). Similar results were observed in CD4⁺ T-cells (Fig. 16A-B).

Overall, TIGIT was exclusively present in T-cells expressing PD-1, consistent with our previous experiment. Furthermore, immune interventions containing αPD-L1 appear to have a greater impact on the expression of PD-1 and TIGIT in CD8⁺ than CD4⁺ T-cells, as no changes in TIGIT or PD-1 expression were observed in CD4⁺ T-cells. Moreover, the significant reduction in TIGIT+ T-cells in αTIGIT may indicate epitope competition reflecting effective blockade rather than modulation of TIGIT expression. However, different clones were used for the TIGIT blockade (10A7 derivate) and flow cytometry (1G9) experiments.

### Example 10 - sIR4+αPD-L1 treatment enhanced degranulation in αCD3 stimulated CD8+ T cells

Then, the inventors characterised the functional CD8+ T cell responses after overnight stimulation of splenocytes in the presence of TCR stimulation (αCD3) and gp33 peptide. The inventors analysed CD8+ T-cell function based on the production of IFNy, CD107a and TNF after receiving 3 and 5 treatment doses on days 28 and 34, respectively.

During αCD3 stimulation, an increase in IFNy production was observed in CD8⁺ T cells with chronic LCMV infection compared to Mock, irrespective of the immune intervention given. Nonetheless, a rising trend in IFNy CD8⁺ T-cells was observed in the presence of αTIGIT and sIR4+αPD-L1 compared to αPD-L1 treated infected mice on day 34 (Fig. 17A). In addition, the inventors detected a significant increase in degranulation in CD8⁺ T-cells in sIR4+αPD-L1 and αTIGIT+αPD-L1 treatment compared with LCMV-infected mice and αPD-L1 treatment on day 34 (Fig. 17A-B). Notably, after background subtraction, the inventors observed a decreased production of TNF in LCMV-infected mice compared to Mock mice on day 28. Additionally, the inventors observed increased TNF production after αCD3 stimulation in CD8⁺ T-cells from single sIR4, slR9 and αTIGIT on day 28. However, the production of TNF was generally lower on day 34 (Fig. 17A-C). Importantly, the inventors did not detect IL-10 and IL-6 in our experimental setting (data not shown).

In summary, the results suggest that the combination of sIR4+αPD-L1 and αTIGIT treatments tend to increase IFNy production in CD8⁺ T-cells after five doses, as compared to single αPD-L1 treatment. This effect was not observed in the control group of LMCV-infected mice, indicating that the combined sIR4+αPD-L1 treatment was not inferior to the single αPD-L1 treatment. Additionally, mice receiving five doses of sIR4+αPD-L1 and αTIGIT+αPD-L1 showed enhanced degranulation of CD8⁺ T-cells, which suggests an increased functionality of CD8 T-cells of combinatorial treatments.

### Example 11 - sIR4+αPD-L1 treatment induces activation of the TNFRI/TNF pathway and production of immunoregulatory cytokines.

The inventors conducted a multiplex assay to evaluate 17 cytokines, chemokines and soluble factors in serum samples collected after 3 and 5 doses. To identify changes in soluble immune mediators associated with the immune interventions, the inventors normalised values based on fold change (FCh) from day 21, before immunotherapy administration. On day 28, the inventors could determine the TNFRI, TNF, IL-5, IL-10, RANTES, MIP1α, and BAFF levels by multiplex assay. Notably, the inventors observed a significant increase in FCh TNFRI between LCMV-infected mice and sIR4+αPD-L1 and αTIGIT-αPD-L1 treated mice on day 28. Additionally, an increase in TNF was detected between LCMV-infected mice and αPD-L1, sIR4+αPD-L1, and sIR9+αPD-L1, as well as an increase in the production of IL-10 between LCMV-infected mice and αTIGIT+αPD-L1 treated mice (Fig. 18A). On day 34, the inventors could only determine the levels of TNFRI, IL-4, IL-5, IL-10, IL-13, MIP1a, and BAFF. Consistent with day 28, the inventors observed an increase in FCh TNFRI between LCMV-infected mice and sIR4+αPD-L1 on day 38. Furthermore, the inventors detected an increase in IL-4 and IL-13 induced by sIR4+αPD-L1 treatment (Fig. 18B).

### Example 12 - Reduction of the kidney damage in the LCMV model by effect of siR4/α-PD-L1 administration

Finally, the inventors analyzed the kidney damage induced by LCMV infection and subsequent treatment. At both endpoints, day 28 and day 34, PD-L1 treatment induced the highest damage score in terms of inflammation in the analysed kidneys.

The combination of PD-L1 with sIRs was able to decrease the damage induced by this treatment in, at least, 2 out 3 mice (Fig. 19).

FASTA The nucleotide and amino acid sequence for all sIR prototypes in FASTA format are provided in the following table (Table 6).

**Table 6. Amino acid sequences of siR prototypes**

| | Human sIR prototypes |
|---|---|
| >sIR1 - Human monomer (SP Nat) | |
| MRWCLLLIWA QGLRQAPLAS GMMTGTIETT GNISAEKGGS IILQCHLSST TAQVTQVNWE QQDQLLAICN ADLGWHISPS FKDRVAPGPG LGLTLQSLTV 100 NDTGEYFCIY HTYPDGTYTG RIFLEVLESS VAEHGARFQI PLEHHHHHH* 150 | |
| >sIR1 - Human monomer (SP CD5) | |
| MPMGSLQPLA TLYLLGMLVA SVLAMMTGTI ETTGNISAEK GGSIILQCHL SSTTAQVTQV NWEQQDQLLA ICNADLGWHI SPSFKDRVAP GPGLGLTLQS 100 LTVNDTGEYF CIYHTYPDGT YTGRIFLEVL ESSGSHHHHH H* 142 | |
| >sIH1 - Human monomer (SP Azu) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS HHHHHH* 137 | |
| >sIR2 - Human dimer (Effector human lgG1) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS KSCDKTHTCP PCPAPELLGG PSVFLFPPKP KDTLMISRTP EVTCVVVDVS HEDPEVKFNW YVDGVEVHNA 200 KTKPREEQYN STYRVVSVLT VLHQDWLNGK EYKCKVSNKA LPAPIEKTIS KAKGQPREPQ VYTLPPSRDE LTKNQVSLTC LVKGFYPSDI AVEWESNGQP 300 ENNYKTTPPV LDSDGSFFLY SKLTVDKSRW QQGNVFSCSV MHEALHNHYT QKSLSLSPGK RSEQKLISEE DLNMHTGHHH HHH* 384 | |
| >sIH10 - Human dimer (No effector human IgG4 S288P) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS ESKYGPPCPP CPAPEFLGGP SVFLFPPKPK DTLMISRTPE VTCVVVDVSQ EDPEVQFNWY VDGVEVHNAK 200 TKPREEQFNS TYRVVSVLTV LHQDWLNGKE YKCKVSNKGL PSSIEKTISK AKGQPREPQV YTLPPSQEEM TKNQVSLTCL VKGFYPSDIA VEWESNGQPE 300 NNYKTTPPVL DSDGSFFLYS RLTVDKSRWQ EGNVFSCSVM HEALHNHYTQ KSLSLSLGKR SEQKLISEED LNMHTGHHHH HH* 383 | |

| Hybrid sIR prototypes | |
|---|---|
| >sIR3 - Hybrid dimer (Effector murine IgG2c) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS RVPITQNPCP PLKECPPCAA PDLLGGPSVF IFPPKIKDVL MISLSPMVTC VVVDVSEDDP DVQISWFVNN 200 VEVHTAQTQT HREDYNSTLR VVSALPIQHQ DWMSGKEFKC KVNNRALPSP IEKTISKPRG PVRAPQVYVL PPPAEEMTKK EFSLTCMITG FLPAEIAVDW 300 TSNGRTEQNY KNTATVLDSD GSYFMYSKLR VQKSTWERGS LFACSVVHEG LHNHLTTKTI SRSLGKDYKD DDDKNMHTGH HHHHH* 386 | |
| >sIR4 - Hybrid dimer (Effector murine IgG2c ΔPCPP) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS RVPITQNΔLKE CPPCAAPDLL GGPSVFIFPP KIKDVLMISL SPMVTCVVVD VSEDDPDVQI SWFVNNVEVH 200 TAQTQTHRED YNSTLRVVSA LPIQHQDWMS GKEFKCKVNN RALPSPIEKT ISKPRGPVRA PQVYVLPPPA EEMTKKEFSL TCMITGFLPA EIAVDWTSNG 300 RTEQNYKNTA TVLDSDGSYF MYSKLRVQKS TWERGSLFAC SVVHEGLHNH LTTKTISRSL GKDYKDDDDK NMHTGHHHHH H* 382 | |
| >sIR9 - Hybrid dimer (No Effector murine IgG1) | |
| MTRLTVLALL AGLLASSRAM MTGTIETTGN ISAEKGGSII LQCHLSSTTA QVTQVNWEQQ DQLLAICNAD LGWHISPSFK DRVAPGPGLG LTLQSLTVND 100 TGEYFCIYHT YPDGTYTGRI FLEVLESSGS VPRDCGCKPC ICTVPEVSSV FIFPPKPKDV LTITLTPKVT CVVVDISKDD PEVQFSWFVD DVEVHTAQTQ 200 PREEQFNSTF RSVSELPIMH QDWLNGKEFK CRVNSAAFPA PIEKTISKTK GRPKAPQVYT IPPPKEQMAK DKVSLTCMIT DFFPEDITVE WQWNGQPAEN300 YKNTQPIMNT NGSYFVYSKL NVQKSNWEAG NTFTCSVLHE GLHNHHTEKS LSHSPGKDYK DDDDKNMHTG HHHHHH* 377 | |

| Murine sIR prototypes | |
|---|---|
| >sIR5 - Murine monomer | |
| MTRLTVLALL AGLLASSRAT IDTKRNISAE EGGSVILQCH FSSDTAEVTQ VDWKQQDQLL AIYSVDLGWH VASVFSDRVV PGPSLGLTFQ SLTMNDTGEY 100 FCTYHTYPGG IYKGRIFLKV QESSGSHHHH HH* 133 | |
| >sIR6 - Murine dimer ( Effector murine IgG2c) | |
| MTRLTVLALL AGLLASSRAT IDTKRNISAE EGGSVILQCH FSSDTAEVTQ VDWKQQDQLL AIYSVDLGWH VASVFSDRVV PGPSLGLTFQ SLTMNDTGEY 100 FCTYHTYPGG IYKGRIFLKV QESSGSRVPI TQNPCPPLKE CPPCAAPDLL GGPSVFIFPP KIKDVLMISL SPMVTCVVVD VSEDDPDVQI SWFVNNVEVH 200 TAQTQTHRED YNSTLRVVSA LPIQHQDWMS GKEFKCKVNN RALPSPIEKT ISKPRGPVRA PQVYVLPPPA EEMTKKEFSL TCMITGFLPA EIAVDWTSNG 300 RTEQNYKNTA TVLDSDGSYF MYSKLRVQKS TWERGSLFAC SVVHEGLHNH LTTKTISRSL GKDYKDDDDK NMHTGHHHHH H* 382 | |
| >sIR7 - Murine dimer ( Effector murine IgG2c ΔPCPP) | |
| MTRLTVLALL AGLLASSRAT IDTKRNISAE EGGSVILQCH FSSDTAEVTQ VDWKQQDQLL AIYSVDLGWH VASVFSDRVV PGPSLGLTFQ SLTMNDTGEY 100 FCTYHTYPGG IYKGRIFLKV QESSGSRVPI TQNΔLKECPPC AAPDLLGGPS VFIFPPKIKD VLMISLSPMV TCVVVDVSED DPDVQISWFV NNVEVHTAQT 200 QTHREDYNST LRVVSALPIQ HQDWMSGKEF KCKVNNRALP SPIEKTISKP RGPVRAPQVY VLPPPAEEMT KKEFSLTCMI TGFLPAEIAV DWTSNGRTEQ 300 NYKNTATVLD SDGSYFMYSK LRVQKSTWER GSLFACSVVH EGLHNHLTTK TISRSLGKDY KDDDDKNMHT GHHHHHH* 378 | |
| >sIR8 - Murine dimer (No Effector murine IgG1) | |
| MTRLTVLALL AGLLASSRAT IDTKRNISAE EGGSVILQCH FSSDTAEVTQ VDWKQQDQLL AIYSVDLGWH VASVFSDRVV PGPSLGLTFQ SLTMNDTGEY 100 FCTYHTYPGG IYKGRIFLKV QESSGSVPRD CGCKPCICTV PEVSSVFIFP PKPKDVLTIT LTPKVTCVVV DISKDDPEVQ FSWFVDDVEV HTAQTQPREE 200 QFNSTFRSVS ELPIMHQDWL NGKEFKCRVN SAAFPAPIEK TISKTKGRPK APQVYTIPPP KEQMAKDKVS LTCMITDFFP EDITVEWQWN GQPAENYKNT 300 QPIMNTNGSY FVYSKLNVQK SNWEAGNTFT CSVLHEGLHN HHTEKSLSHS PGKDYKDDDD KNMHTGHHHH HH* 373 | |

| | |
|---|---|
| SP Azu (bold black); Homodimerization domain bold black underline); VTQ/AX6G/TYP domain (gray); BamHl GS linker; ΔPCPP deletion; Myc-tag (Cyan); Flag-tag (underline); His-Tag (HHHHH); Stop Codon (*) | |

## Claims

1. A composition comprising:
a) a first component selected from the group consisting of:
i) a polypeptide comprising a TIGIT variant that comprises the TIGIT Ig-like V-type domain and wherein said polypeptide lacks the TIGIT transmembrane and cytoplasmic domains and,
ii) a polynucleotide encoding the polypeptide defined in i),
and
b) a second component, which is an inhibitor of the PD-1/PD-L1 axis.

2. The composition according to claim 1 wherein the TIGIT variant essentially consists of amino acids 22 to 124 of human TIGIT, wherein the numbering is as defined in the NCBI database entry (Release of 10 March 2024) under accession number NP_776160 or of amino acids 20 to 119 of the mouse TIGIT wherein the numbering is as defined in the NCBI database entry (Release of 24 May 2024) under accession number NP_001139797.

3. The composition according to claim 2 wherein the TIGIT variant is a polypeptide as defined in SEQ ID NO: 1 or SEQ ID NO: 39 or SEQ ID NO: 2.

4. The composition according to claim 1 wherein the first component is a fusion protein comprising the TIGIT variant and a non-TIGIT polypeptide region or a polynucleotide encoding said fusion protein.

5. The composition according to claim 4 wherein the TIGIT variant essentially consists of amino acids 41 to 122 of human TIGIT, wherein the numbering is as defined in the NCBI database entry (Release of 10 March 2024) under accession number NP_776160 or of amino acids 38 to 119 of the mouse TIGIT wherein the numbering is as defined in the NCBI database entry (Release of 24 May 2024) under accession number NP_001139797.

6. The composition according to claims 4 or 5 wherein the non-TIGIT polypeptide region is a Fc monomer which is formed by the hinge region and the CH2 and CH3 domains of an IgG molecule wherein preferably the Fc monomer is selected from the group consisting of the human lgG1 Fc monomer, the human IgG1 Fc monomer carrying a S228P mutation, the mouse IgG2c Fc monomer, the mouse IgG2c Fc monomer carrying the ΔPCPP mutation and the mouse IgG1 Fc monomer.

7. The composition according to claim 6 wherein the Fc monomer is selected from the group consisting of SEQ ID NO: 3, 4, 5, 6 or 7.

8. The composition according to claim 7 wherein the TIGIT variant is selected from the group consisting of:
a) A fusion protein comprising the human TIGIT Ig-like V-type domain and a human IgG1 Fc monomer,
b) A fusion protein comprising the human TIGIT Ig-like V-type domain and a human IgG4 Fc monomer carrying a S228P mutation,
c) A fusion protein comprising the mouse TIGIT Ig-like V-type domain and a mouse !gG2c Fc monomer,
d) A fusion protein comprising the mouse TIGIT Ig-like V-type domain and a mouse IgG2c Fc monomer carrying a ΔPCPP mutation,
e) A fusion protein comprising the mouse TIGIT Ig-like V-type domain and a mouse IgG1 Fc monomer,
f) A fusion protein comprising the human TIGIT Ig-like V-type domain and a mouse !gG2c Fc monomer,
g) A fusion protein comprising the human TIGIT Ig-like V-type domain and a mouse IgG2c Fc monomer carrying a ΔPCPP mutation and
h) A fusion protein comprising the human TIGIT Ig-like V-type domain and a mouse IgG1 Fc monomer.

9. The composition according to claim 8 wherein the fusion protein is selected from the group consisting of SEQ ID NO: 8 to 15.

10. The composition according to any of claims 1 to 9, wherein the inhibitor of the PD-1/PD-L1 axis is selected from the group consisting of a PD-1 binding antagonist, a PD-L1 binding antagonist and a PD-L2 binding antagonist.

11. The composition according to claim 10 wherein the first component is the polypeptide as defined in SEQ ID NO: 14 and the second component is an anti-PD-L1 antibody or wherein the first component is the polypeptide as defined in SEQ ID NO: 15 and the second component is an anti-PD-L1 antibody.

12. The composition according to any one of claims 1 to 11 for use in a method for treating or delaying an immune-related disease.

13. The composition or pharmaceutical composition for use according to claim 12 wherein the immune related disease is a chronic viral infection, a chronic bacterial infection, and a tumor.

14. A polypeptide comprising a TIGIT variant that comprises the TIGIT Ig-like V-type domain and wherein said polypeptide lacks the TIGIT transmembrane and cytoplasmic domains or a polynucleotide encoding said polypeptide for use in a method for reducing immune toxicity in a patient that is being treated with an inhibitor of the PD-1/PD-L-1 axis.

15. The polypeptide or polynucleotide for use according to claim 14 wherein the polypeptide or polynucleotide is as defined in component (i) of the composition according to any of claims 1 to 12.
